# EUROPEAN PATENT SPECIFICATION

(11) **EP 3 178 922 B1**
(45) Date of publication and mention of the grant of the patent: **22.05.2019**
(21) Application number: 16202662.9
(22) Date of filing: 07.12.2016
(51) Int. Cl.: C12N 1/20, C12N 9/02, C12N 9/04, C12N 15/31, C12N 15/70

(54) **BACTERIAL CYTOCHROME P450 PROTEIN VARIANT AND METHOD OF REDUCING CONCENTRATION OF FLUORINATED METHANE IN SAMPLE USING THE SAME**
BAKTERIELLE CYTOCHROM-P450-PROTEINVARIANTE UND VERFAHREN ZUR VERRINGERUNG DER KONZENTRATION VON FLUORIERTEM METHAN IN EINER PROBE DAMIT
VARIANTE DE PROTÉINE P450 CYTOCHROME BACTÉRIENNE ET PROCÉDÉ DE RÉDUCTION DE LA CONCENTRATION DE MÉTHANE FLUORÉ DANS UN ÉCHANTILLON À L'AIDE DE CELLE-CI

(30) Priority: 07.12.2015 KR 20150173293; 21.04.2016 KR 20160048960; 17.06.2016 KR 20160075831; 26.08.2016 KR 20160109543; 26.08.2016 KR 20160109544
(43) Date of publication of application: 14.06.2017
(73) Proprietor: Samsung Electronics Co., Ltd., Gyeonggi-do 16677 (KR)
(72) Inventor: JUNG, Yukyung, Suwon-si 16678 (KR); YANG, Dongsik, Suwon-si 16678 (KR); PARK, Jinhwan, Suwon-si 16678 (KR); KIM, Taeyong, Suwon-si 16678 (KR); KANG, Changduk, Suwon-si 16678 (KR); BHADURI, Anirban, 560037 Bangalore (IN); SIVA KUMAR, Tadi Venkata, 560037 Bangalore (IN)
(74) Representative: Grünecker Patent- und Rechtsanwälte PartG mbB

(56) References cited:
- WO-A2-2008/016709
- US-B1- 6 794 168
- J.-Q. LIU ET AL: "Reaction Mechanism of Fluoroacetate Dehalogenase from Moraxella sp. B", JOURNAL OF BIOLOGICAL CHEMISTRY, vol. 273, no. 47, 20 November 1998 (1998-11-20), pages 30897-30902, XP055306169, US ISSN: 0021-9258, DOI: 10.1074/jbc.273.47.30897

## Description

### BACKGROUND

### 1. Field

The present disclosure relates to a recombinant microorganism including an exogenous gene encoding a bacterial cytochrome P450 protein (in the following also referred to as P450, recombinant P450 or recombinant P450 protein), a composition including the recombinant P450 protein, which is used for removing fluorinated methane represented by CHₙF₄₋ₙ (n is an integer of 0 to 3) in a sample, and a method of reducing a concentration of CHₙF₄₋ₙ in the sample.

### 2. Description of the Related Art

The emissions of greenhouse gases which have accelerated global warming are one of the serious environmental problems, and regulations to reduce and prevent the emissions of greenhouse gases have been tightened. Among the greenhouse gases, fluorinated gases (F-gas) such as perfluorocarbons (PFCs), hydrofluorocarbons (HFCs), and sulfur hexafluoride (SF₆) show low absolute emission, but have a long half-life and a very high global warming potential, resulting in significant adverse environmental impacts. The amount of F-gas emitted from semiconductor and electronics industries, which are major causes of F-gas emission, has exceeded the assigned amount of greenhouse gas emissions and continues to increase. Therefore, costs required for degradation of greenhouse gases and greenhouse gas emission allowances are increasing every year.

A pyrolysis or catalytic thermal oxidation process has been generally used in the decomposition of F-gas. However, this process has disadvantages of limited decomposition rate, emission of secondary pollutants, high cost, etc. To help solve this problem, biological decomposition of F-gas using a microbial biocatalyst has been adopted. Nevertheless, there remains a need for new methods and compositions for removing fluorinated methanes.
WO 2008/016709 A2 refers to a method and system for selectively fluorinating organic molecules on a target site wherein the target site is activated and then fluorinated. In one embodiment, the target side is oxidized by an oxidizing agent, e.g. a P450 oxygenase. P450_{BM3} from *Bacillus megaterium* as well as P450_{CAM} from *Pseudomonas putida* are disclosed.
US 6,794,168 B1 to the process of oxidising a substrate which is a halo aromatic compound, with a monooxygenase enzyme. The enzyme may be P450_{CAM}. The process may be carried out in cells, animals or plants. Variants of P450_{CAM} or P450_{BM3} are mentioned.

### SUMMARY

An aspect provides a recombinant microorganism including an exogenous gene encoding a bacterial cytochrome P450 protein variant as defined in claim 1.

Another aspect provides a composition for removing fluorinated methane represented by CHₙF₄₋ₙ (n is an integer of 0 to 3) in a sample, as defined in claim 5,.

Still another aspect provides a method of reducing a concentration of CHₙF₄₋ₙ in a sample as defined in claim 9.

Still another aspect provides the variant of bacterial cytochrome P450 protein as defined in claim 4 and a polynucleotide encoding the same as defined in claim 14.

### BRIEF DESCRIPTION OF THE DRAWINGS

These and/or other aspects will become apparent and more readily appreciated from the following description of the embodiments, taken in conjunction with the accompanying drawings in which:
FIG. 1 shows a vector map of a pETDuet-camC-camAB vector;
FIG. 2 shows changes in a headspace concentration of CHF₃ over time when *E. coli* BL21/pETDuet-camC-camAB was cultured in a medium contacted with CHF₃-containing gas;
FIG. 3A shows changes in a headspace concentration of CHCl₃ over time when *E. coli* BL21/pETDuet-camC-camAB was cultured in a CHCl₃-containing medium;
FIG. 3B shows changes in a headspace concentration of CF₄ over time when *E. coli* BL21/pETDuet-camC-camAB was cultured in a medium contacted with CF₄-containing gas;
FIG. 3C shows changes of CF₄ in a sample over time by *E. coli* BL21/pETDuet-camCmt-camAB introduced with a mutant camC gene;
FIG. 4 shows a vector map of a pET28a-P450_{BM3} vector;
FIG. 5 shows a vector map of a pACYCDuet-zwf vector;
FIG. 6 shows changes in a headspace concentration of CHF₃ over time when recombinant *E. coli* BL21/pET28a-P450_{BM3} or recombinant *E. coli* BL21/pET28a-P450_{BM3}+pACYCDuet-zwf was cultured in a solution contacted with CHF₃-containing gas;
FIG. 7 shows changes in a headspace concentration of CHCl₃ over time when *E. coli* BL21/pET28a-P450_{BM3} was cultured in a CHCl₃-containing solution;
FIG. 8 shows changes in a headspace concentration of CF₄ over time when *E. coli* BL21/pET28a-P450_{BM3} was cultured for 7 days in a medium contacted with CF₄-containing gas; and
FIG. 9 shows changes in a concentration of CF₄ in a sample over time by *E. coli* BL21/pET28a-P450BM3mt introduced with a mutant P450BM3 gene.

### DETAILED DESCRIPTION

An aspect provides a recombinant microorganism including an exogenous gene encoding a bacterial cytochrome P450 protein variant as defined in claim 1.

Cytochromes P450 (CYPs) belong to the superfamily of proteins containing a heme cofactor, and therefore, are hemoproteins. Cytochromes P450 use a variety of small and large molecules as substrates in enzymatic reactions. They are, in general, terminal oxidase enzymes in electron transfer chains, broadly categorized as P450-containing systems.

Bacterial cytochromes P450 are often soluble enzymes and are involved in diverse metabolic processes. Some bacteria such *E. coli* have no cytochrome P450. Cytochrome P450 (CYP101) derived from *P. putida* is part of a camphor-hydroxylating catalytic cycle consisting of two electron transfer steps from putidaredoxin, which is a 2Fe-2S cluster-containing protein cofactor.

Cytochrome P450BM3 (CYP102) derived from *B. megaterium* catalyzes the NADPH-dependent hydroxylation of several long-chain fatty acids at the ω-1 through ω-3 positions. Cytochrome P450BM3 constitutes a natural fusion protein between the CYP domain and an electron donating cofactor.

With regard to the recombinant microorganism, the cytochrome P450 protein variant is P450Cam or P450BM3.

P450Cam may be derived from *Pseudomonas putida* PpG786. P450BM3 may be derived from *Bacillus megaterium* (ATCC 14581). The cytochrome P450Cam protein may be a complex of CamA, CamB, and CamC, which constitutes the bacterial CYP101 system. CamA may be FAD-containing reductase. CamA may be NADH or NADPH-dependent. The CamA may belong to EC 1.18.1.5. CamB may be [2Fe2S]-type ferredoxin. CamC, also called P450Cam (CYP101), may include cytochrome P450 and may belong to EC 1.14.15.1. CamA, CamB, and CamC may have amino acid sequences of SEQ ID NOS: 2, 4, and 6, respectively. Genes encoding CamA, CamB, and CamC may have nucleotide sequences of SEQ ID NOS: 1, 3, and 5, respectively.

The P450Cam variant has an amino acid alteration at an amino acid residue corresponding to position F351 of an amino acid sequence of SEQ ID NO: 6, and has an activity belonging to EC 1.14.15.1. The amino acid alteration is replacement of the amino acid residue corresponding to position F351 with Y, T, N, Q, H, or D (e.g., a F351Y, F351T, F351N, F351Q, F351H, or F351D variant). EC 1.14.15.1 may represent an enzyme that catalyzes the reaction of (+)-camphor+reduced putidaredoxin + O₂ ⇔ (+)-exo-5-hydroxycamphor + oxidized putidaredoxin + H₂O.

A gene encoding the P450Cam variant is a gene encoding the F351Y, F351T, F351N, F351Q, F351H, or F351D variant in P450Cam having the amino acid sequence of SEQ ID NO: 6. The gene may have a nucleotide sequence of SEQ ID NO: 51, 52, 53, 54, 55, or 56, or corresponding sequence by virtue of the degeneracy of the genetic code (e.g., a codon-optimized sequence). The microorganism may further include a gene encoding CamA and a gene encoding CamB.

P450BM3 may be a polypeptide having an amino acid sequence of SEQ ID NO: 8. A gene encoding P450BM3 may have a nucleotide sequence of SEQ ID NO: 7. The variant has an amino acid alteration at an amino acid residue corresponding to the position N320 of the amino acid sequence of SEQ ID NO: 8, and has an activity belonging to EC 1.14.14.1. The variant has replacement of the amino acid residue corresponding to the position N320 of SEQ ID NO: 8 with W, F, G, P, S, or E (e.g., a N320W, N320F, N320G, N320P, N320S, or N320E variant). A gene encoding the P450BM3 variant is a gene encoding the variant having a N320W, N320F, N320G, N320P, N320S, or N320E substation in P450BM3 having the amino acid sequence of SEQ ID NO: 8. The gene may have a nucleotide sequence of SEQ ID NO: 45, 46, 47, 48, 49, or 50, or corresponding sequence by virtue of the degeneracy of the genetic code (e.g., a codon-optimized sequence)..

"EC 1.14.14.1" may catalyze the following reaction: RH + [reduced NADPH-hemoprotein reductase] + O₂ ⇔ ROH + [oxidized NADPH---hemoprotein reductase] + H₂O.

As used herein, the term "corresponding" refers to the amino acid position of a protein of interest that aligns with the mentioned position (e.g., position F351 of SEQ ID NO: 6 or position N320 of SEQ ID NO: 8) of a reference protein when amino acid sequences of the protein of interest and the reference protein are aligned using an art-acceptable protein alignment program, including the NCBI BLAST pairwise alignment or the well known Lipman-Pearson Protein Alignment program, with the following parameters: Ktuple=2, Gap Penalty=4, and Gap length penalty=12. In this regard, the range included in the "corresponding" sequence may be a range of E-value 0.00001 and H-value 0.001.

Examples of proteins homologs of P450Cam with an amino acid substitution at a position corresponding to position F351 of SEQ ID NO: 6, obtained according to the above alignment conditions, are listed in the following Tables 1, 2, and 3.

Also, examples of homologs of P450BM3 with an amino acid substitution at a position corresponding to position N320 of SEQ ID NO: 8, obtained according to the above alignment conditions, are listed in the following Tables 4 and 5.

The P450 variant can comprise SEQ ID NO: 6 with the described substitution at F351 of SEQ ID NO: 6, or can comprise SEQ ID NO: 8. In some embodiments, the p450 variant comprises an amino acid sequence with at least 75, 80, 85, 90, 91, 93, 94, 95, 95, 97, 98, or 99% sequence identity to SEQ ID NO: 6 or SEQ ID NO: 8, including the amino acid alteration at F351 of SEQ ID NO: 6 or N320 of SEQ ID NO: 8. Also contemplated are fragments (e.g., N or C terminal truncations or internal deletions) that retain the recited activity.

**[Table 1]**

| NO. | NCBI ID | NO. | NCBI ID | NO. | NCBI ID |
|---|---|---|---|---|---|
| 1 | gi\|163930960 | 31 | gi\|551338874 | 61 | gi\|728824802 |
| 2 | gi\|612182735 | 32 | gi\|730289226 | 62 | gi\|746238551 |
| 3 | gi\|497125935 | 33 | gi\|654615031 | 63 | gi\|494955160 |
| 4 | gi\|310942843 | 34 | gi\|656116930 | 64 | gi\|496104589 |
| 5 | gi\|657832383 | 35 | gi\|826046029 | 65 | gi\|567412687 |
| 6 | gi\|498088271 | 36 | gi\|737567226 | 66 | gi\|98976470 |
| 7 | gi\|544829275 | 37 | gi\|98976439 | 67 | gi\|358240604 |
| 8 | gi\|861974080 | 38 | gi\|612108073 | 68 | gi\|494898237 |
| 9 | gi\|738620841 | 39 | gi\|515116019 | 69 | gi\|504740033 |
| 10 | gi\|499763441 | 40 | gi\|817101463 | 70 | gi\|648547795 |
| 11 | gi\|746290673 | 41 | gi\|398136480 | 71 | gi\|808659667 |
| 12 | gi\|503614840 | 42 | gi\|757698965 | 72 | gi\|551292036 |
| 13 | gi\|861969570 | 43 | gi\|126194726 | 73 | gi\|551292470 |
| 14 | gi\|662139213 | 44 | gi\|737644672 | 74 | gi\|502616812 |
| 15 | gi\|544827262 | 45 | gi\|496199226 | 75 | gi\|545316934 |
| 16 | gi\|498088269 | 46 | gi\|654614522 | 76 | gi\|491842667 |
| 17 | gi\|861974085 | 47 | gi\|759387698 | 77 | gi\|750353906 |
| 18 | gi\|737512009 | 48 | gi\|739341634 | 78 | gi\|858007594 |
| 19 | gi\|817101596 | 49 | gi\|783098869 | 79 | gi\|665981585 |
| 20 | gi\|494439068 | 50 | gi\|783093808 | 80 | gi\|806833869 |
| 21 | gi\|496309894 | 51 | gi\|783094059 | 81 | gi\|504197805 |
| 22 | gi\|746289514 | 52 | gi\|759427060 | 82 | gi\|564970689 |
| 23 | gi\|753796069 | 53 | gi\|861968800 | 83 | gi\|499924508 |
| 24 | gi\|545454562 | 54 | gi\|497509581 | 84 | gi\|765348796 |
| 25 | gi\|648417306 | 55 | gi\|497810537 | 85 | gi\|497424876 |
| 26 | gi\|666681698 | 56 | gi\|783098865 | 86 | gi\|739367531 |
| 27 | gi\|567402060 | 57 | gi\|739341585 | 87 | gi\|517897868 |
| 28 | gi\|826044703 | 58 | gi\|783097592 | 88 | gi\|702839727 |
| 29 | gi\|826049125 | 59 | gi\|783099978 | 89 | gi\|739883834 |
| 30 | gi\|806905723 | 60 | gi\|783097681 | 90 | gi\|750419370 |

**[Table 2]**

| NO. | NCBI ID | NO. | NCBI ID | NO. | NCBI ID |
|---|---|---|---|---|---|
| 91 | gi\|494019567 | 121 | gi\|783096369 | 151 | gi\|518973859 |
| 92 | gi\|498089540 | 122 | gi\|530258733 | 152 | gi\|808102361 |
| 93 | gi\|826041014 | 123 | gi\|657922982 | 153 | gi\|664078266 |
| 94 | gi\|566044904 | 124 | gi\|654614517 | 154 | gi\|663221595 |
| 95 | gi\|566044935 | 125 | gi\|739616950 | 155 | gi\|663326563 |
| 96 | gi\|501296495 | 126 | gi\|783098022 | 156 | gi\|493424288 |
| 97 | gi\|517247251 | 127 | gi\|806908467 | 157 | gi\|502993954 |
| 98 | gi\|516019877 | 128 | gi\|21467173 | 158 | gi\|518949456 |
| 99 | gi\|288913356 | 129 | gi\|401808868 | 159 | gi\|750417303 |
| 100 | gi\|512734904 | 130 | gi\|514397583 | 160 | gi\|750543392 |
| 101 | gi\|783100677 | 131 | gi\|749201634 | 161 | gi\|377530614 |
| 102 | gi\|764994003 | 132 | gi\|528182079 | 162 | gi\|377532462 |
| 103 | gi\|517247290 | 133 | gi\|647795133 | 163 | gi\|737965741 |
| 104 | gi\|652940833 | 134 | gi\|651636595 | 164 | gi\|739538229 |
| 105 | gi\|169821418 | 135 | gi\|661269250 | 165 | gi\|739543125 |
| 106 | gi\|835531786 | 136 | gi\|695263348 | 166 | gi\|750407524 |
| 107 | gi\|551362264 | 137 | gi\|696511194 | 167 | gi\|739645497 |
| 108 | gi\|764960072 | 138 | gi\|493217416 | 168 | gi\|493919745 |
| 109 | gi\|759387686 | 139 | gi\|493377553 | 169 | gi\|737792710 |
| 110 | gi\|737512189 | 140 | gi\|563565380 | 170 | gi\|750519055 |
| 111 | gi\|820802680 | 141 | gi\|665834124 | 171 | gi\|521297725 |
| 112 | gi\|646534628 | 142 | gi\|665888884 | 172 | gi\|482632482 |
| 113 | gi\|820802864 | 143 | gi\|665827329 | 173 | gi\|494797766 |
| 114 | gi\|334103741 | 144 | gi\|751294725 | 174 | gi\|493993588 |
| 115 | gi\|826041595 | 145 | gi\|827106327 | 175 | gi\|648280499 |
| 116 | gi\|746241621 | 146 | gi\|639165413 | 176 | gi\|750519223 |
| 117 | gi\|764993875 | 147 | gi\|652899356 | 177 | gi\|498814706 |
| 118 | gi\|748599849 | 148 | gi\|652914977 | 178 | gi\|519015945 |
| 119 | gi\|764997807 | 149 | gi\|652694819 | 179 | gi\|639007804 |
| 120 | gi\|662354752 | 150 | gi\|496153669 | 180 | gi\|518767974 |

**[Table 3]**

| NO. | NCBI ID | NO. | NCBI ID | NO. | NCBI ID |
|---|---|---|---|---|---|
| 181 | gi\|739625293 | 213 | gi\|652908779 | 245 | gi\|499912932 |
| 182 | gi\|820802866 | 214 | gi\|503298839 | 246 | gi\|657825087 |
| 183 | gi\|739651753 | 215 | gi\|740869740 | 247 | gi\|655586613 |
| 184 | gi\|502742128 | 216 | gi\|503612867 | 248 | gi\|739190742 |
| 185 | gi\|515118033 | 217 | gi\|646519758 | 249 | gi\|518714103 |
| 186 | gi\|820802677 | 218 | gi\|494981163 | 250 | gi\|503189844 |
| 187 | gi\|391860290 | 219 | gi\|490214493 | 251 | gi\|739186131 |
| 188 | gi\|737643185 | 220 | gi\|736859678 | 252 | gi\|739186149 |
| 189 | gi\|544823238 | 221 | gi\|739577671 | 253 | gi\|516607102 |
| 190 | gi\|763095543 | 222 | gi\|736886954 | 254 | gi\|522116265 |
| 191 | gi\|739611016 | 223 | gi\|654534319 | 255 | gi\|522150263 |
| 192 | gi\|145322598 | 224 | gi\|549129549 | 256 | gi\|703225980 |
| 193 | gi\|825391797 | 225 | gi\|653383901 | 257 | gi\|703223632 |
| 194 | gi\|759685456 | 226 | gi\|703388673 | 258 | gi\|703223663 |
| 195 | gi\|836723496 | 227 | gi\|653777500 | 259 | gi\|494300956 |
| 196 | gi\|488703345 | 228 | gi\|655968891 | 260 | gi\|808659227 |
| 197 | gi\|763384158 | 229 | gi\|655882347 | 261 | gi\|489969104 |
| 198 | gi\|528059914 | 230 | gi\|630947972 | 262 | gi\|806822276 |
| 199 | gi\|783097229 | 231 | gi\|495218410 | 263 | gi\|556618018 |
| 200 | gi\|494017068 | 232 | gi\|768967538 | 264 | gi\|738609029 |
| 201 | gi\|739663478 | 233 | gi\|746229913 | 265 | gi\|403646243 |
| 202 | gi\|739620206 | 234 | gi\|746230981 | 266 | gi\|737785331 |
| 203 | gi\|746237691 | 235 | gi\|746236533 | 267 | gi\|703226655 |
| 204 | gi\|567412712 | 236 | gi\|544823589 | 268 | gi\|602519307 |
| 205 | gi\|550925359 | 237 | gi\|746239269 | 269 | gi\|739367513 |
| 206 | gi\|746344573 | 238 | gi\|490319630 | 270 | gi\|737980497 |
| 207 | gi\|530255704 | 239 | gi\|494981649 | 271 | gi\|737981631 |
| 208 | gi\|739669024 | 240 | gi\|494957004 | 272 | gi\|817101442 |
| 209 | gi\|654478200 | 241 | gi\|763090173 | 273 | gi\|497809551 |
| 210 | gi\|490753280 | 242 | gi\|738613213 | 274 | gi\|545453717 |
| 211 | gi\|497922631 | 243 | gi\|746229737 | 275 | gi\|497809089 |
| 212 | gi\|740896970 | 244 | gi\|754958228 | | |

**[Table 4]**

| NO. | NCBI ID | NO. | NCBI ID | NO. | NCBI ID |
|---|---|---|---|---|---|
| 1 | gi\|515136080 | 30 | gi\|491699287 | 59 | gi\|518088806 |
| 2 | gi\|757757972 | 31 | gi\|518251998 | 60 | gi\|768926886 |
| 3 | gi\|822528663 | 32 | gi\|493730772 | 61 | gi\|498013687 |
| 4 | gi\|544838284 | 33 | gi\|817723893 | 62 | gi\|498020927 |
| 5 | gi\|491696887 | 34 | gi\|228697407 | 63 | gi\|498487619 |
| 6 | gi\|655149838 | 35 | gi\|228736549 | 64 | gi\|530665825 |
| 7 | gi\|512150124 | 36 | gi\|692165489 | 65 | gi\|753200845 |
| 8 | gi\|493729782 | 37 | gi\|489315595 | 66 | gi\|495633284 |
| 9 | gi\|738856821 | 38 | gi\|498015014 | 67 | gi\|748815403 |
| 10 | gi\|655112080 | 39 | gi\|749037577 | 68 | gi\|738932691 |
| 11 | gi\|648634781 | 40 | gi\|763303489 | 69 | gi\|738896417 |
| 12 | gi\|522106669 | 41 | gi\|830323790 | 70 | gi\|652405427 |
| 13 | gi\|504462655 | 42 | gi\|857573616 | 71 | gi\|764371274 |
| 14 | gi\|783152040 | 43 | gi\|654951198 | 72 | gi\|701527930 |
| 15 | gi\|759010788 | 44 | gi\|647569946 | 73 | gi\|751587021 |
| 16 | gi\|545381104 | 45 | gi\|738784028 | 74 | gi\|736758744 |
| 17 | gi\|548617766 | 46 | gi\|515717624 | 75 | gi\|657859536 |
| 18 | gi\|648623486 | 47 | gi\|517613324 | 76 | gi\|657039097 |
| 19 | gi\|738714376 | 48 | gi\|507035289 | 77 | gi\|852221735 |
| 20 | gi\|639453808 | 49 | gi\|661257874 | 78 | gi\|850337075 |
| 21 | gi\|497281073 | 50 | gi\|655116131 | 79 | gi\|550547409 |
| 22 | gi\|494207912 | 51 | gi\|736161405 | 80 | gi\|495772021 |
| 23 | gi\|843075790 | 52 | gi\|493687687 | 81 | gi\|504454491 |
| 24 | gi\|518517905 | 53 | gi\|806498422 | 82 | gi\|737572351 |
| 25 | gi\|655094715 | 54 | gi\|532550849 | 83 | gi\|654483633 |
| 26 | gi\|517805393 | 55 | gi\|757435944 | 84 | gi\|495911896 |
| 27 | gi\|518469404 | 56 | gi\|737448097 | 85 | gi\|737423431 |
| 28 | gi\|655084756 | 57 | gi\|542116840 | 86 | gi\|737423433 |
| 29 | gi\|764415731 | 58 | gi\|764608412 | | |

The recombinant microorganism may be bacteria or fungi. The bacteria may be Gram-positive or Gram-negative bacteria. The Gram-negative bacteria may belong to the family *Enterobacteriaceae.* The Gram-negative bacteria may belong to the genus *Escherichia,* the genus *Samonella*, the genus *Xanthomonas,* or the genus *Pseudomonas.* The genus *Escherichia* microorganism may be *E. coli.* The genus *Xanthomonas* microorganism may include *Xanthobacter autotrophicus.* Gram-positive bacteria may belong to the genus *Corynebacterium* or the genus *Bacillus.*

The recombinant microorganism may have a genetic modification that increases the level (activity or protein level) of an enzyme that catalyzes a NADPH production reaction to increase an intracellular NADPH level by the reaction. The genetic modification can be amplification of an endogenous gene or introduction of an exogenous gene. The enzyme may be a protein belonging to EC 1.1.1.49. The enzyme may be glucose-6-phosphate dehydrogenase (G6PD or G6PDH). The recombinant microorganism may further include an exogenous gene encoding G6PDH.

Another aspect provides a composition including the recombinant microorganism and/or the recombinant P450 protein variant which is useful for removing a halogenated methane such as fluorinated methane represented by CHₙF₄₋ₙ (n is an integer of 0 to 3) in a sample. Unless otherwise specified, the recombinant P450 protein variant is the same as described above.

With regard to the composition, fluorinated methane represented by CHₙF₄₋ₙ may be, CHF₃, CH₂F₂, CH₃F, or CF₄. The term "removing" includes reducing of a concentration of fluorinated methane in the sample. The reducing includes complete removal.

With regard to the composition, the recombinant P450 protein variant may be in a recombinant microorganism, or the composition can comprise a lysate thereof, or a water-soluble material fraction of the lysate. When in a recombinant microorganism, the bacterial cytochrome P450 variant may be expressed from an exogenous gene.

The recombinant microorganism may be bacteria or fungi. The bacteria may be Gram-positive or Gram-negative bacteria. The Gram-negative bacteria may belong to the family *Enterobacteriaceae.* The Gram-negative bacteria may belong to the genus *Escherichia,* the genus *Samonella*, the genus *Xanthomonas,* or the genus *Pseudomonas.* The genus *Escherichia* microorganism may be *E. coli.* The genus *Xanthomonas* microorganism may include *Xanthobacter autotrophicus.* Gram-positive bacteria may belong to the genus *Corynebacterium* or the genus *Bacillus.*

Removing fluorinated methane may include cleaving of C-F bonds of fluorinated methane, converting of fluorinated methane into other materials, or reducing of the concentration of fluorinated methane in the sample by intracellular accumulation. The converting may be introducing of a hydrophilic group such as a hydroxyl group into fluorinated methane or introducing of a carbon-carbon double bond or a carbon-carbon triple bond thereto.

With regard to the composition, the sample may be in a liquid or gas state. The sample may be industrial waste water or waste gas.

Still another aspect provides a method of reducing a concentration of fluorinated methane in a sample; the method includes contacting
a) a recombinant microorganism comprising an exogenous gene encoding a bacterial cytochrome P450 protein or a variant thereof, wherein the variant is a P450Cam variant having an amino acid alteration at an amino acid residue corresponding to the position F351 of an amino acid sequence of SEQ ID NO: 6 and having an activity belonging to EC 1.14.15.1 or a P450BM3 variant having an amino acid alteration at an amino acid residue corresponding to the position N320 of an amino acid sequence of SEQ ID NO: 8 and having an activity belonging to EC 1.14.14.1, and/or
b) the recombinant microorganism of any one of Claims 1 to 3 and 6 to 8, a lysate thereof or a water-soluble fraction of the lysate, and/or
c) the variant of bacterial P450Cam of claim 3, and/or
d) the variant of bacterial P450BM3 of Claim 3, and/or
e) the composition of any one of Claims 5 to 8,
with the sample containing fluorinated methane represented by CHₙF₄₋ₙ (n is an integer of 0 to 3) to reduce the concentration of fluorinated methane in the sample. Unless otherwise specified, the recombinant P450 protein variant is the same as described above.

Contacting of the recombinant P450 protein variant with the sample may be performed in a sealed container. The contacting may be gas-liquid contact of contacting a gas sample with a liquid containing the recombinant P450 protein variant. Further, the contacting may be liquid-liquid contact of contacting a liquid sample with a liquid containing the recombinant P450 protein variant. The liquid-liquid contact includes mixing thereof.

With regard to the method, the recombinant P450 protein variant may be in a recombinant microorganism that expresses bacterial cytochrome P450 protein, or a lysate thereof or the water-soluble material fraction of the lysate, or the recombinant P450 protein itself (e.g., isolated protein).

The contacting may be performed in the sealed container under conditions where the recombinant microorganism may survive or be viable. The conditions where the recombinant microorganism may survive or be viable may be conditions where the recombinant microorganism may be allowed to proliferate or to be in a resting state. In this case, the contacting may be culturing of the microorganism in the presence of fluorinated methane. The culturing may be performed under aerobic or anaerobic conditions.

The recombinant microorganism may be bacteria or fungi. The bacteria may be Gram-positive or Gram-negative bacteria. The Gram-negative bacteria may belong to the family *Enterobacteriaceae.* The Gram-negative bacteria may belong to the genus *Escherichia,* the genus *Samonella*, the genus *Xanthomonas,* or the genus *Pseudomonas.* The genus *Escherichia* microorganism may be *E. coli.* The genus *Xanthomonas* microorganism may include *Xanthobacter autotrophicus.* Gram-positive bacteria may belong to the genus *Corynebacterium* or the genus *Bacillus.*

With regard to the method, the sample may be in a liquid or gas state. The sample may be industrial waste water or waste gas.

Still another aspect provides the variant of bacterial cytochrome P450 protein and a polynucleotide encoding the same.

The variant may be as described above. In one embodiment, the variant has an amino acid alteration at an amino acid residue corresponding to position F351 of an amino acid sequence of SEQ ID NO: 6, and has an activity belonging to EC 1.14.15.1. The variant has replacement of the amino acid residue at position F351 with Y, T, N, Q, H or D, in camC of P450Cam having the amino acid sequence of SEQ ID NO: 6. The variant may be a F351Y, F351T, F351N, F351Q, F351H, or F351D mutant in camC of P450Cam having the amino acid sequence of SEQ ID NO: 6. In another embodiment, the variant may have an amino acid alteration at an amino acid residue corresponding to position N320 of an amino acid sequence of SEQ ID NO: 8, and has an activity belonging to EC 1.14.14.1. The variant has replacement of the amino acid residue at the position N320 with W, T, G, P, S, or E in P450BM3 having the amino acid sequence of SEQ ID NO: 8. The variant may be N320W, N320F, N320G, N320P, N320S, or N320E in P450BM3 having the amino acid sequence of SEQ ID NO: 8.

The polynucleotide encoding the variant can be codon optimized for use in various organisms. The polynucleotide encoding the variant may be included in a vector. The vector may be any vector, as long as it is used to introduce the polynucleotide into microorganisms. The vector may be a plasmid or viral vector. The polynucleotide may be operably linked to suitable regulatory sequences.

The recombinant microorganism according to an aspect may be used for removing fluorinated methane represented by CHₙF₄₋ₙ (or other halogenated methane) in the sample.

The variant of the recombinant P450 protein according to an aspect may be used for removing fluorinated methane in the sample.

The composition including the recombinant P450 protein variant according to another aspect may be used for removing fluorinated methane in the sample.

The method of reducing the concentration of fluorinated methane in the sample according to still another aspect may efficiently reduce the concentration of fluorinated methane in the sample. For example, a headspace concentration of fluorinated methane can be reduced, by at least 3, 4, 5, 10, 15, or 20% when measured according to the protocol of any of the Examples below. The activity of the p450 variant can be a multiple of 1.5, 2, 2.5, 3, 3.5, 4, or greater, of the wild-type enzyme (*in vitro* or in an otherwise genetically identical strain).

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects.

Hereinafter, the present invention will be described in more detail with reference to Examples. However, these Examples are for illustrative purposes only, and the scope of the present invention is not intended to be limited by these Examples.

### Example 1: Recombinant E. coli expressing P450Cam gene and Removal of halomethane in sample by using the same

In this Example, a recombinant *E*. *coli* expressing a P450Cam gene was prepared, and an effect of removing halomethane, i.e., CHF₃, CF₄, or CHCl₃ in a sample by using the same was examined.

### (1) Preparation of recombinant E. coli expressing P450_{CAM} gene

As P450Cam genes, camC, camA, and camB genes were amplified from CAM plasmid of *Pseudomonas putida* PpG786 strain, respectively. camC, camA, and camB genes have nucleotide sequences of SEQ ID NO: 5, SEQ ID NO: 1, and SEQ ID NO: 3, respectively. These genes encode amino acid sequences of SEQ ID NO: 6, SEQ ID NO: 2, and SEQ ID NO: 4, respectively. In detail, *P. putida* PpG786 strain DSM 7162 was cultured in an LB medium at 30°C under stirring at 230 rpm overnight, and then CAM plasmid was isolated using a total DNA extraction kit (Invitrogen Biotechnology). PCR was performed using the CAM plasmid as a template and a set of primers having nucleotide sequences of SEQ ID NOS: 11 and 12; a set of primers having nucleotide sequences of SEQ ID NOS: 13 and 14; and a set of primers having nucleotide sequences of SEQ ID NOS: 15 and 16 to amplify and obtain camA, camB, and camC genes, respectively.

The camC gene which was amplified by PCR using a set of primers of nucleotide sequences of SEQ ID NOS: 11 and 12 was ligated with pETDuet (Novagen, Cat. No.71146-3), which was digested with restriction enzymes, NcoI and HindII, using an InFusion Cloning Kit (Clontech Laboratories, Inc.) to prepare a pETDuet-camC vector. Further, the prepared pETDuet-camC vector was digested with restriction enzymes, Ndel and XhoI, and ligated with the amplified camA and the amplified camB gene fragment using the InFusion Cloning Kit (Clontech Laboratories, Inc.) to prepare a pETDuet-camC-camAB vector.

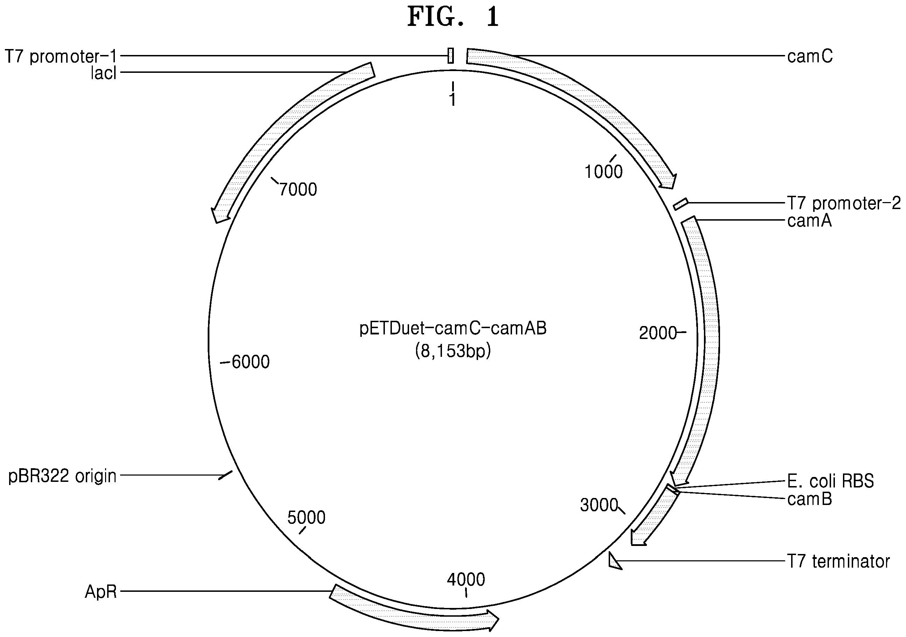

FIG. 1 shows a vector map of the pETDuet-camC-camAB vector.

Next, *E. coli* BL21 strain was introduced with the prepared pETDuet-camC-camAB vector by a heat shock method, and then cultured on a LB plate containing 100 µg/mL of ampicillin. A strain showing ampicillin resistance was selected. Finally, the strain thus selected was designated as a recombinant *E. coli* BL21/pETDuet-camC-camAB.

### (2) Effect of removing CHF₃ or CHCl₃ in sample by recombinant E. coli expressing P450Cam gene

In this section, it was examined whether the P450_{CAM} gene-introduced, *E. coli* BL21/pETDuet-camC-camAB strain prepared in section (1) affects removal of CHF₃ or CHCl₃ in a sample. In detail, *E. coli* BL21/pETDuet-camC-camAB was cultured in a TB medium at 30°C under stirring at 230 rpm. At OD₆₀₀ of about 0.5, 0.5 mM of IPTG was added thereto, followed by culturing at 25°C and 230 rpm overnight. The cells were harvested and suspended in an M9 medium to a cell density of OD₆₀₀ of 2.5. 10 ml of this cell suspension was added to a 60 ml-serum bottle, and then the bottle was sealed. The terrific broth (TB) medium included 12 g of tryptone, 24 g of yeast extract, 5 g of glycerol, and 89 mM phosphate buffer per 1 L of distilled water. Further, the M9 medium included 6 g of Na₂HPO₄, 3 g of KH₂PO₄, 0.5 g of NaCl, and 1 g of NH₄Cl per 1 L of distilled water.

Next, gas-phase CHF₃ was injected through a rubber stopper of a cap of the serum bottle using a syringe to its headspace concentration of 200 ppm. Further, liquid-phase CHCl₃ was injected through the rubber stopper of the cap of the serum bottle using the syringe to its concentration of 0.02 mM in the medium. Thereafter, the serum bottle was incubated for 18 hrs to 152 hrs, while stirring at 30°C and 200 rpm. Each experiment was performed in triplicate.

At a predetermined time interval during incubation, 0.5 ml of the headspace gas containing no medium in the serum bottle was collected using a 1.0 ml-headspace syringe and injected into GC (Agilent 7890, Palo Alto, CA, USA). The injected CHF₃ or CHCl₃ was separated through a CP-PoraBOND Q column (25 m length, 0.32 mm i.d., 5 um film thickness, Agilent), and changes in the CHF₃ or CHCl₃ concentration were analyzed by mass spectrometry (Agilent 5973, Palo Alto, CA, USA). As a carrier gas, helium was used, and applied to the column at a flow rate of 1.5 ml/min. GC conditions were as follows: An inlet temperature was 250°C, an initial temperature was maintained at 40°C for 2 minutes, and temperature was raised to 290°C at a rate of 20°C/min. MS conditions were as follows: Ionization energy was 70 eV, an interface temperature was 280°C, an ion source temperature was 230°C, and a quadrupole temperature was 150°C.

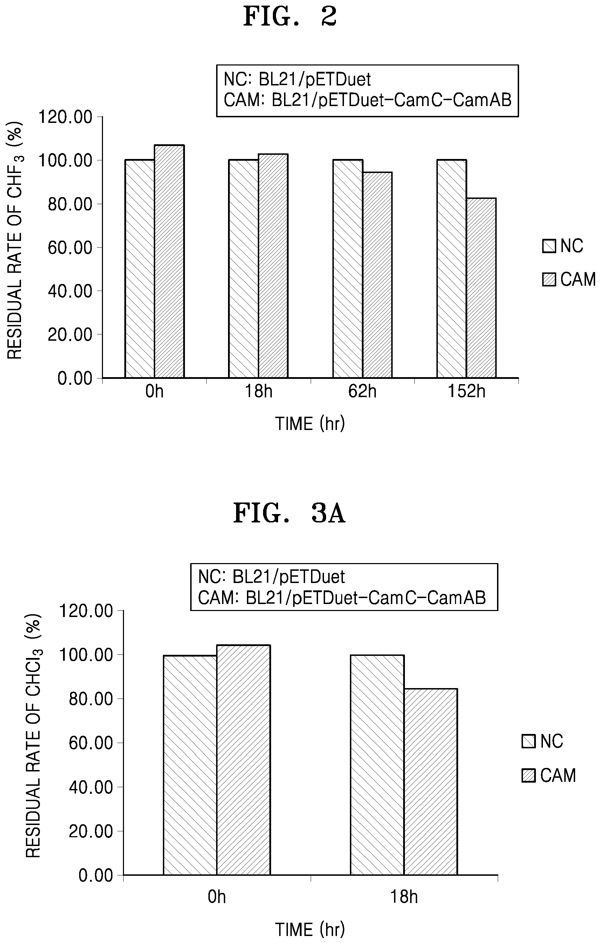

FIG. 2 shows changes in a headspace concentration of CHF₃ over time when *E. coli* BL21/pETDuet-camC-camAB was cultured in the medium contacted with CHF₃-containing gas.

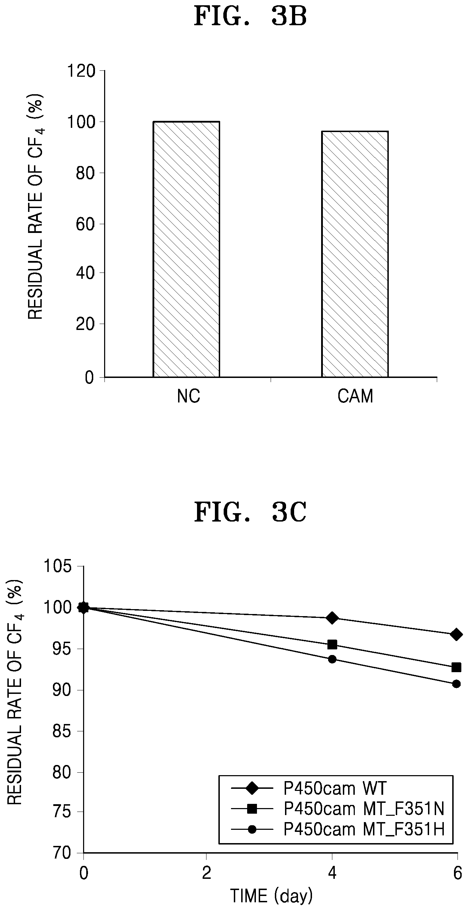

FIG. 3A shows changes in a headspace concentration of CHCl₃ over time when *E. coli* BL21/pETDuet-camC-camAB was cultured in a CHCl₃-containing medium. In FIGS. 2, 3A and 3B, NC represents a negative control group, and 'CAM' represents an experiment performed by using *E. coli* BL21/pETDuet-camC-camAB. As shown in FIG. 2, when the *E. coli* BL21/pETDuet-camC-camAB was cultured for 62 hours and 152 hours, the headspace concentration of CHF₃ was decreased by about 5.6% and about 17.3%, respectively, compared to the control group. Further, as shown in FIG. 3A, when the *E. coli* BL21/pETDuet-camC-camAB was cultured for 18 hours, the headspace concentration of CHCl₃ was decreased by about 14.8%, compared to the control group.

### (3) Effect of removing CF₄ in sample by recombinant E. coli expressing P450Cam gene

In this section, it was examined whether the P450_{CAM} gene-introduced, *E. coli* BL21/pETDuet-camC-camAB strain prepared in section (1) affects removal of CF₄ in a sample.

The experiment was performed in the same manner as the procedure performed for CHF₃ in Section (2), except that CF₄ was used instead of CHF₃ and gas-phase CF₄ was injected through a rubber stopper of a cap of the serum bottle using a syringe to its headspace concentration of 1000 ppm, and then the serum bottle was incubated for 7 days, while stirring at 30°C and 200 rpm. The results are as shown in FIG. 3B.

FIG. 3B shows changes in a headspace concentration of CF₄ over time when *E. coli* BL21/pETDuet-camC-camAB was cultured in a medium contacted with CF₄-containing gas. As shown in FIG. 3B, when the *E. coli* BL21/pETDuet-camC-camAB was cultured for 7 days, the headspace concentration of CF₄ was decreased by about 3.57%, compared to the control group.

### (4) Recombinant E. coli expressing mutant P450Cam gene and Effect of removing CF₄ in sample thereby

In this section, mutants were prepared in order to improve the activity of removing fluorinated methane in a sample by P450Cam. Phenylalanine (hereinafter, referred to as "F351") at position 351 of the amino acid sequence of SEQ ID NO: 6 was replaced by other 19 natural amino acids (hereinafter, referred to as "F351X". Here, X represents 19 natural amino acids other than phenylalanine), and each of the genes encoding the mutants was introduced into *E. coli,* and their activity of removing CF₄ in a sample was examined. camC corresponds to heme domain and F351 is one of conserved amino acids in the amino acid sequences of camC derived from many different species.

### (4.1) Preparation of 19 mutants

Preparation of the F351X mutants of SEQ ID NO: 6 was performed using a QuikChange II Site-Directed Mutagenesis Kit (Agilent Technology, USA). Site-directed mutagenesis using the kit was performed using *PfuUltra* high-fidelity (HF) DNA polymerase for mutagenic primer-directed replication of both plasmid strands with the highest fidelity. The basic procedure utilized a supercoiled double-stranded DNA (dsDNA) vector with an insert of interest and two synthetic oligonucleotide primers, both containing the desired mutation. The oligonucleotide primers, each complementary to opposite strands of the vector, were extended during temperature cycling by *PfuUltra* HF DNA polymerase, without primer displacement. Extension of the oligonucleotide primers generated a mutated plasmid containing staggered nicks. Following temperature cycling, the product was treated with *Dpn I.* The *Dpn I* endonuclease (target sequence: 5'-Gm⁶ATC-3') was specific for methylated and hemimethylated DNA and was used to digest the parental DNA template and to select for mutation-containing synthesized DNA. The nicked vector DNA incorporating the desired mutations was then transformed into XL1-Blue supercompetent cells. The sequence identifiers for the primer sets used to produce the mutations are given in the following Table 6.

**[Table 6]**

| NO. | Mutation type | Primer sequence |
|---|---|---|
| 1 | F351Y | SEQ ID NOS: 21 and 22 |
| 2 | F351T | SEQ ID NOS: 23 and 24 |
| 3 | F351N | SEQ ID NOS: 25 and 26 |
| 4 | F351Q | SEQ ID NOS: 27 and 28 |
| 5 | F351H | SEQ ID NOS: 29 and 30 |
| 6 | F351D | SEQ ID NOS: 31 and 32 |

In detail, PCR was performed using the pETDuet-camC-camAB vector prepared in (1) as a template and each of the primer sets described in Table 6 as a primer and *PfuUlta* HF DNA polymerase to obtain mutated vectors. These vector products were treated with *Dpnl* to select mutation-containing synthesized DNAs. The vector DNA incorporating the desired mutations was then transformed into XL1-Blue supercompetent cells to clone a pETDuet-camCmt-camAB vector.

Lastly, the cloned pETDuet-camCmt-camAB vector and pETDuet-camCwt-camAB vector were introduced into *E. coli* BL21 strain in the same manner as in (1), and a finally selected strain was designated as recombinant *E. coli* BL21/pETDuet-camCmt-camAB.

### (4.2) Effect of removing CF₄ in sample by recombinant E. coli BL21/pETDuet-camCmt-camAB

In this section, it was examined whether the mutant camC gene-introduced, *E. coli* BL21/pETDuet-camCmt-camAB prepared in section (4.1) affects removal of CF₄ in a sample.

The experiment was performed in the same manner as the procedure performed for CHF₃ in Section (2), except that CF₄ was used instead of CHF₃ and gas-phase CF₄ was injected through a rubber stopper of a cap of the serum bottle using a syringe to a headspace concentration of 1000 ppm, and then the serum bottle was incubated for 6 days, while stirring at 30°C and 230 rpm. The results are as shown in Table 7.

**[Table 7]**

| NO. | Mutation type | Residual amount of CF₄ (Percentage relative to control group) | Reduction rate of CF₄ (Percentage relative to control group) |
|---|---|---|---|
| 1 | F351Y | 91.82 | 8.18 |
| 2 | F351T | 95.42 | 4.58 |
| 3 | F351N | 92.56 | 7.44 |
| 4 | F351Q | 94.12 | 5.88 |
| 5 | F351H | 89.85 | 10.15 |
| 6 | F351D | 94.31 | 5.69 |
| 7 | F351* | 96.43 | 3.57 |

In Table 7, the control group represents *E. coli* introduced with the pETDuet vector instead of the pETDuet-camCmt-camAB vector, and F351* represents wild-type camC.

Further, in this section, the experiment was performed in the same manner as the procedure performed for CHF₃ in Section (2), except that 20 mL of mutant camC-introduced *E. coli* BL21/pETDuet-camCmt-camAB (OD₆₀₀=3.0) prepared in Section (4.1) was injected to a 175-mL flask, CF₄ was used instead of CHF₃, and gas-phase CF₄ was injected through a rubber stopper of a cap of the serum bottle using a syringe to a headspace concentration of 1000 ppm, and then the serum bottle was incubated for 6 days, while stirring at 30°C and 230 rpm. A residual amount of CF₄ over time, that is, a remaining percentage (%) of CF₄ was examined. The results are shown in FIG. 3C.

FIG. 3C shows changes of CF₄ in a sample over time by *E. coli* BL21/pETDuet-camCmt-camAB introduced with the mutant camC gene. As shown in FIG. 3C, when the recombinant *E*. *coli* P450CAM strain, namely, F351N or F351H mutant gene-containing strain was cultured for 6 days, the CF₄ level was further decreased by about 7.02% or about 8.92%, compared to the control group. In contrast, the wild-type strain further decreased the CF₄ level by about 3.14%, compared to the control group.

### Example 2: Recombinant E. coli expressing P450BM3 gene and Removal of halomethane in sample by using the same

In this Example, a recombinant *E. coli* expressing a P450BM3 gene was prepared, and an effect of removing halomethane, i.e., CHF₃, CF₄, or CHCl₃ in a sample by using the same was examined.

### (1) Preparation of recombinant E. coli expressing P450BM3 gene

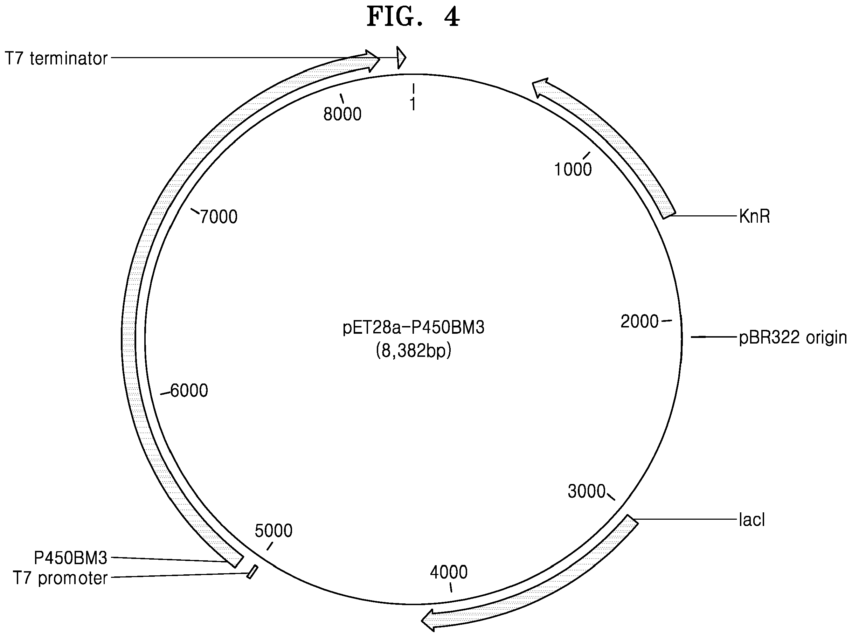

P450_{BM3} gene of *Bacillus megaterium* (ATCC 14581) strain was amplified. P450_{BM3} gene has a nucleotide sequence of SEQ ID NO: 7, and encodes an amino acid sequence of SEQ ID NO: 8. In detail, *B. megaterium* (ATCC 14581) was cultured in an LB medium at 30°C under stirring at 230 rpm overnight, and then a genomic DNA was isolated using the total DNA extraction kit (Invitrogen Biotechnology). PCR was performed using this genomic DNA as a template and a set of primers having nucleotide sequences of SEQ ID NOS: 17 and 18 to amplify and obtain the P450BM3 gene. The P450BM3 gene thus amplified was ligated with pET28a (Novagen, Cat. No.69864-3), which was digested with restriction enzymes, Ncol and Xhol, using the InFusion Cloning Kit (Clontech Laboratories, Inc.) to prepare a pET28a-P450BM3 vector. FIG. 4 shows a vector map of the pET28a-P450BM3 vector.

Further, in order to increase an intracellular NADPH level, a zwf gene encoding glucose 6-phosphate dehydrogenase of *E. coli* K12 (MG1655) was amplified. The Zwf gene has a nucleotide sequence of SEQ ID NO: 9, and encodes an amino acid sequence of SEQ ID NO: 10. In detail, *E. coli* was cultured in an LB medium at 37°C under stirring at 230 rpm overnight, and then a genomic DNA was isolated using the total DNA extraction kit (Invitrogen Biotechnology). PCR was performed using this genomic DNA as a template and a set of primers having nucleotide sequences of SEQ ID NOS: 19 and 20 to amplify and obtain the zwf gene. The zwf gene thus amplified was ligated with pACYCDuet (Novagen, Cat. No. 71147-3), which was digested with restriction enzymes, Ncol and Sacl, using the InFusion Cloning Kit (Clontech Laboratories, Inc.) to prepare a pACYCDuet-zwf vector.

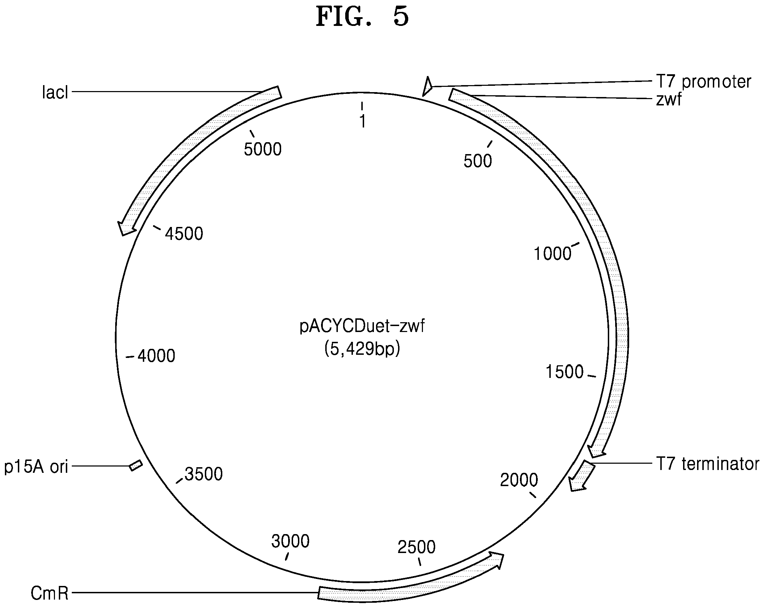

FIG. 5 shows a vector map of the pACYCDuet-zwf vector.

Next, *E. coli* BL21 strain was introduced with the prepared pET28a-P450_{BM3} vector by a heat shock method, and then cultured on a LB plate containing 50 µg/mL of kanamycin. A strain showing kanamycin resistance was selected. Finally, the strain thus selected was designated as a recombinant *E. coli* BL21/pET28a-P450BM3.

Further, *E. coli* BL21 strain was introduced with the prepared pET28a-P450_{BM3} vector and pACYCDuet-zwf vector by a heat shock method, and then cultured on a LB plate containing 50 µg/mL of kanamycin and 35 µg/mL of chloramphenicol. A strain showing kanamycin resistance and chloramphenicol resistance was selected. Finally, the strain thus selected was designated as a recombinant *E. coli* BL21/pET28a-P450_{BM3}+pACYCDuet-zwf.

### (2) Effect of removing CHF₃ or CHCl₃ in sample by recombinant E. coli expressing P450BM3 gene

In this section, it was examined whether the P450BM3 gene-introduced, recombinant *E. coli* BL21/pET28a-P450BM3 strain or BL21/pET28a-P450BM₃+pACYCDuet-zwf strain prepared in section (1) affects removal of CHF₃ or CHCl₃ in a sample.

In detail, *E. coli* BL21/pET28a-P450_{BM3} or BL21/pET28a-P450BM3+pACYCDuet-zwf strain was cultured in the TB medium at 30°C under stirring at 230 rpm. At OD₆₀₀ of about 0.5, 0.2 mM of IPTG was added thereto, followed by culturing at 25°C and 230 rpm overnight. The cells were harvested and suspended in the M9 medium to a cell density of OD₆₀₀ of 2.5. 10 ml of this cell suspension was added to a 60 ml-serum bottle, and then the bottle was sealed. The TB medium and the M9 medium are the same as those described in Example 1.

Next, gas-phase CHF₃ was injected through a rubber stopper of a cap of the serum bottle using a syringe to a headspace concentration of 200 ppm. Further, liquid-phase CHCl₃ was injected through the rubber stopper of the cap of the serum bottle using the syringe to its concentration of 0.02 mM in the medium. Thereafter, the serum bottle was incubated for 15 hrs to 142 hrs, while stirring at 30°C and 230 rpm. Each experiment was performed in triplicate.

At a predetermined time interval during incubation, the headspace concentration of CHF₃ or CHCl₃ in the serum bottle was analyzed under the same conditions as described in (2) of Example 1.

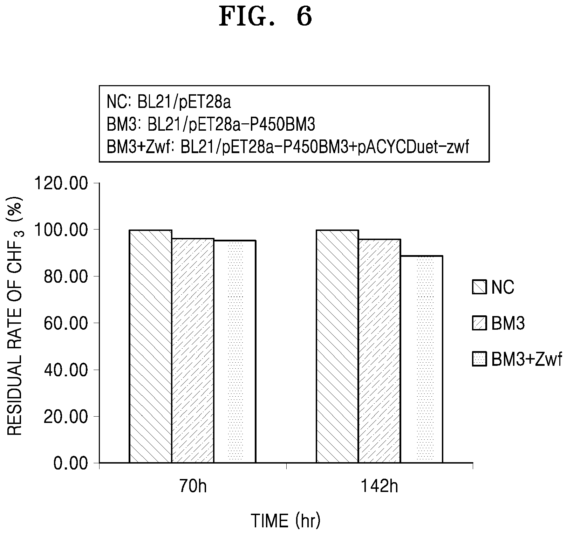

FIG. 6 shows changes in headspace concentration of CHF₃ over time when *E. coli* BL21/pET28a-P450BM3 or BL21/pET28a-P450BM3+pACYCDuet-zwf was cultured for 142 hours in a medium contacted with CHF₃-containing gas.

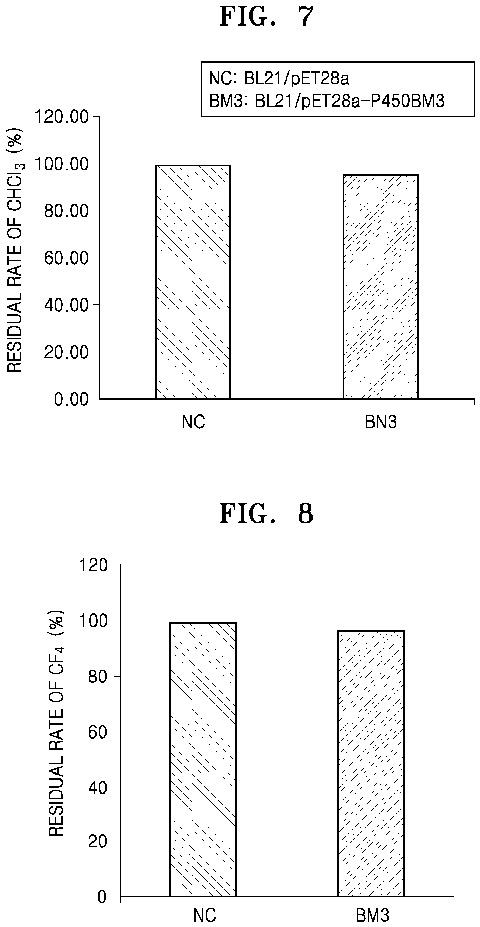

FIG. 7 shows changes in headspace concentration of CHCl₃ over time when *E. coli* BL21/pET28a-P450BM3 was cultured for 15 hours in a CHCl₃-containing medium. In FIGS. 6, 7, and 8, NC represents a negative control group, 'BM3' represents an experiment performed by using *E. coli* BL21/pET28a-P450BM3, and 'BM3+Zwf' represents an experiment performed by using *E. coli* BL21/pET28a-P450BM3+pACYCDuet-zwf. As shown in FIG. 6, when the *E. coli* BL21/pET28a-P450BM3 and *E. coli* BL21/pET28a-P450BM3+pACYCDuet-zwf were cultured for 70 hours and 142 hours, the headspace concentration of CHF₃ was decreased, compared to the control group, by about 3.93% and about 4.57% upon culturing for 70 hours and by about 4.15% and about 11.03% upon culturing for 142 hours, respectively. Further, as shown in FIG. 7, when they were cultured for 15 hours, the headspace concentration of CHCl₃ was decreased by about 4.1%, compared to the control group.

### (3) Effect of removing CF₄ in sample by recombinant E. coli expressing P450BM3 gene

In this section, it was examined whether the P450BM3 gene-introduced, *E. coli* BL21/pET28a-P450BM3 strain prepared in section (1) affects removal of CF₄ in a sample.

The experiment was performed in the same manner as the procedure performed for CHF₃ in Section (2), except that CF₄ was used instead of CHF₃ and gas-phase CF₄ was injected through a rubber stopper of a cap of the serum bottle using a syringe to its headspace concentration of 1000 ppm, and then the serum bottle was incubated for 7 days, while stirring at 30°C and 200 rpm. The results are as shown in FIG. 8.

FIG. 8 shows changes in a headspace concentration of CF₄ over time when *E. coli* BL21/pET28a-P450BM3 was cultured for 7 days in a medium contacted with CF₄-containing gas. As shown in FIG. 8, when the *E. coli* BL21/pET28a-P450BM3 was cultured for 7 days, the headspace concentration of CF₄ was decreased by about 3.03%, compared to the control group.

### (4) Recombinant E. coli expressing mutant P450_{BM3} gene and Effect of removing CF₄ in sample thereby

In this section, mutants were prepared in order to improve the activity of removing fluorinated methane in a sample by P450BM3. Asparagine (hereinafter, referred to as "N320") at position 320 of the amino acid sequence of SEQ ID NO: 8 was replaced by other 19 natural amino acids (hereinafter, referred to as "N320X". Here, X represents 19 natural amino acids other than asparagine), and each of the genes encoding the mutants was introduced into *E. coli*, and their activity of removing CF₄ in a sample was examined. N320 is included in the heme-containing P450 oxygenase domain, and N320 is one of conserved amino acids in the amino acid sequences of enzymes having the same function.

### (4.1) Preparation of 19 mutants

Preparation of the N320X mutants of SEQ ID NO: 8 was performed using a QuikChange II Site-Directed Mutagenesis Kit (Agilent Technology, USA). Mutagenesis using the kit was performed in the same manner as described above.

Of respective primer sets used to induce N320X mutation, primer sets regarding to the increased activity of removing fluorinated methane in a sample, compared to that of the wild-type *E. coli*, are given in the following Table 8.

**[Table 8]**

| NO. | Mutation type | Primer sequence |
|---|---|---|
| 1 | N320W | SEQ ID NOS: 33 and 34 |
| 2 | N320F | SEQ ID NOS: 35 and 36 |
| 3 | N320G | SEQ ID NOS: 37 and 38 |
| 4 | N320P | SEQ ID NOS: 39 and 40 |
| 5 | N320S | SEQ ID NOS: 41 and 42 |
| 6 | N320E | SEQ ID NOS: 43 and 44 |

In detail, PCR was performed using the pET28a-P450BM3 vector prepared in (1) as a template and each of the primer sets described in Table 8 as a primer and *PfuUlta* HF DNA polymerase to obtain mutated vectors. These vector products were treated with *Dpnl* to select mutation-containing synthesized DNAs. The vector DNA incorporating the desired mutations was then transformed into XL1-Blue supercompetent cells to clone a pET28a-P450BM3mt vector.

Lastly, the cloned pET28a-P450BM3 vector and pET28a-P450BM3mt vector were introduced into *E. coli* BL21 strain in the same manner as in (1), and a finally selected strain was designated as recombinant *E. coli* BL21/pET28a-P450BM3mt.

### (4.2) Effect of removing CF₄ in sample by recombinant E. coli BL21/pET28a-P450BM3mt

In this section, it was examined whether the mutant P450BM3mt-introduced, *E. coli* BL21/pET28a-P450BM3mt prepared in section (4.1) affects removal of CF₄ in a sample.

The experiment was performed in the same manner as the procedure performed for CHF₃ in Section (2), except that CF₄ was used instead of CHF₃ and gas-phase CF₄ was injected through a rubber stopper of a cap of the serum bottle using a syringe to a headspace concentration of 1000 ppm, and then the serum bottle was incubated for 6 days, while stirring at 30°C and 230 rpm. The results are as shown in Table 9.

**[Table 9]**

| NO. | Mutation type | Residual amount of CF₄ (Percentage relative to control group) | Reduction rate of CF₄ (Percentage relative to control group) |
|---|---|---|---|
| 1 | N320W | 94.42 | 5.58 |
| 2 | N320F | 87.38 | 12.62 |
| 3 | N320G | 89.82 | 10.18 |
| 4 | N320P | 86.89 | 13.11 |
| 5 | N320S | 82.03 | 17.97 |
| 6 | N320E | 88.48 | 11.52 |
| 7 | N320* | 96.97 | 3.03 |

In Table 9, the control group represents *E. coli* introduced with the pET28a vector instead of the pET28a-P450BM3mt vector, and N320* represents wild-type P450BM3.

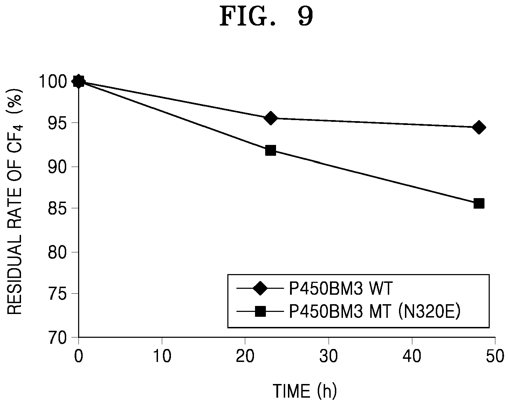

Further, in this section, the experiment was performed in the same manner as the procedure performed for CHF₃ in Section (2), except that 100 mL of mutant P450BM3-introduced *E. coli* BL21/pET28a-P450BM3mt (OD₆₀₀=3.0) prepared in Section (4.1) was injected to a 250-mL flask, CF₄ was used instead of CHF₃, and gas-phase CF₄ was injected through a rubber stopper of a cap of the serum bottle using a syringe to its headspace concentration of 1000 ppm, and then the serum bottle was incubated for 48 hours, while stirring at 30°C and 230 rpm. Culturing was performed in the same manner as for CHF₃, and a residual amount of CF₄ over time, that is, a remaining percentage (%) of CF₄ was examined. The results are shown in FIG. 9.

FIG. 9 shows changes of CF₄ in a sample over time by *E. coli* BL21/pET28a-P450BM3mt introduced with the mutant P450BM3 gene. As shown in FIG. 9, when the recombinant *E. coli* P450BM3 strain, namely, N320E mutant gene-containing strain was cultured for 48 hours, the CF₄ level was further decreased by about 14.3%, compared to the control group. In contrast, the wild-type strain further decreased the CF₄ level by about 5.5%, compared to the control group.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.
## Claims

1. A recombinant microorganism comprising an exogenous gene encoding a bacterial cytochrome P450 protein variant, wherein the variant is a P450Cam variant having an amino acid alteration at an amino acid residue corresponding to the position F351 of an amino acid sequence of SEQ ID NO: 6 and having an activity belonging to EC 1.14.15.1 or a P450BM3 variant having an amino acid alteration at an amino acid residue corresponding to the position N320 of an amino acid sequence of SEQ ID NO: 8 and having an activity belonging to EC 1.14.14.1, wherein the amino acid alteration is replacement of the amino acid residue corresponding to the position F351 with Y, T, N, Q, H, or D, or replacement of the amino acid residue corresponding to the position N320 with W, F, G, P, S, or E.

2. The recombinant microorganism of Claim 1, further comprising an exogenous gene encoding a protein belonging to EC 1.1.1.49.

3. The recombinant microorganism of Claim 2, wherein the exogenous gene encodes glucose-6-phosphate dehydrogenase.

4. A variant of bacterial P450Cam having an amino acid alteration at an amino acid residue corresponding to the position F351 of an amino acid sequence of SEQ ID NO: 6 and having activity belonging to EC 1.14.15.1 or a variant of bacterial P450BM3 having an amino acid alteration at an amino acid residue corresponding to the position N320 of an amino acid sequence of SEQ ID NO: 8 and having activity belonging to EC 1.14.14.1, wherein the amino acid alteration is replacement of the amino acid residue corresponding to the position F351 with Y, T, N, Q, H, or D, or replacement of the amino acid residue corresponding to the position N320 with W, F, G, P, S, or E.

5. A composition for removing fluorinated methane represented by CHₙF₄₋ₙ, wherein n is an integer of 0 to 3, in a sample, said composition comprising the recombinant microorganism of any one of Claims 1 to 3, a lysate thereof or a water-soluble fraction of the lysate, and/or the variant of bacterial P450Cam of claim 4, and/or the variant of bacterial P450BM3 of Claim 4.

6. The recombinant microorganism of any one of Claims 1 to 3, or the composition of Claim 5, wherein the microorganism is a Gram-positive or Gram-negative bacteria.

7. The recombinant microorganism or the composition of Claim 6, wherein the Gram-negative bacteria belongs to the family *Enterobacteriaceae.*

8. The recombinant microorganism or the composition of Claim 6, wherein the Gram-negative bacteria belongs to the genus *Escherichia,* the genus *Samonella,* the genus *Xanthomonas,* or the genus *Pseudomonas,* or wherein the Gram-positive bacteria belongs to the genus *Corynebacterium* or the genus *Bacillus.*

9. A method of reducing a concentration of fluorinated methane in a sample, the method comprising contacting
a) a recombinant microorganism comprising an exogenous gene encoding a bacterial cytochrome P450 protein or a variant thereof, wherein the variant is a P450Cam variant having an amino acid alteration at an amino acid residue corresponding to the position F351 of an amino acid sequence of SEQ ID NO: 6 and having an activity belonging to EC 1.14.15.1 or a P450BM3 variant having an amino acid alteration at an amino acid residue corresponding to the position N320 of an amino acid sequence of SEQ ID NO: 8 and having an activity belonging to EC 1.14.14.1, and/or
b) the recombinant microorganism of any one of Claims 1 to 3 and 6 to 8, a lysate thereof or a water-soluble fraction of the lysate, and/or
c) the variant of bacterial P450Cam of claim 4, and/or
d) the variant of bacterial P450BM3 of Claim 4, and/or
e) the composition of any one of Claims 5 to 8,
with the sample comprising fluorinated methane represented by CHnF4-n, wherein n is an integer of 0 to 3, to reduce the concentration of fluorinated methane in the sample.

10. The method of Claim 9 wherein the contacting is performed in a sealed container.

11. The method of Claim 10, wherein the method comprises contacting the recombinant microorganism of any one of Claims 1 to 3, 6 to 8 and item a) of Claim 9 with the sample and wherein the contacting is performed in a sealed container under conditions where the recombinant microorganism survives or is viable.

12. The method of any one of Claims 9 to 11, wherein the contacting is culturing of the recombinant microorganism of any one of Claims 1 to 3, 6 to 8 and item a) of Claim 9 in the presence of fluorinated methane.

13. The method of any one of Claims 9 to 12, wherein the sample is in a liquid or gas state, wherein the sample preferably is industrial waste water or waste gas.

14. A polynucleotide encoding a variant of bacterial P450Cam having an amino acid alteration at an amino acid residue corresponding to the position F351 of an amino acid sequence of SEQ ID NO: 6 and belonging to EC 1.14.15.1 or a polynucleotide encoding a variant of bacterial P450BM3 having an amino acid alteration at an amino acid residue corresponding to the position N320 of an amino acid sequence of SEQ ID NO: 8 and belonging to EC 1.14.14.1, wherein the amino acid alteration is replacement of the amino acid residue corresponding to the position F351 with Y, T, N, Q, H, or D, or replacement of the amino acid residue corresponding to the position N320 with W, F, G, P, S, or E.

15. A vector comprising the polynucleotide of claim 14.

## Patentansprüche

1. Rekombinanter Mikroorganismus, umfassend ein exogenes Gen, das eine Variante eines bakteriellen Cytochrom P450 Proteins kodiert, wobei es sich bei der Variante um eine P450Cam-Variante handelt, die eine Aminosäureveränderung an einem Aminosäurerest aufweist, welcher der Position F351 einer Aminosäuresequenz der SEQ ID NO: 6 entspricht, und eine zu EC 1.14.15.1 gehörende Aktivität aufweist oder um eine P450BM3-Variante, die eine Aminosäureveränderung an einem Aminosäurerest aufweist, welcher der Position N320 einer Aminosäuresequenz der SEQ ID NO: 8 entspricht, und eine zu EC 1.14.14.1 gehörende Aktivität aufweist, wobei es sich bei der Aminosäureveränderung um den Austausch des Aminosäurerests, welcher der Position F351 entspricht, gegen Y, T, N, Q, H oder D oder den Austausch des Aminosäurerests, welcher der Position N320 entspricht, gegen W, F, G, P, S oder E handelt.

2. Rekombinanter Mikroorganismus nach Anspruch 1, ferner umfassend ein exogenes Gen, das ein zu EC 1.1.1.49 gehörendes Protein kodiert.

3. Rekombinanter Mikroorganismus nach Anspruch 2, wobei das exogene Gen Glucose-6-Phosphatdehydrogenase kodiert.

4. Variante des bakteriellen P450Cam, die eine Aminosäureveränderung an einem Aminosäurerest aufweist, welcher der Position F351 einer Aminosäuresequenz der SEQ ID NO: 6 entspricht, und eine zu EC 1.14.15.1 gehörende Aktivität aufweist, oder Variante des bakteriellen P450BM3, die eine Aminosäureveränderung an einem Aminosäurerest aufweist, welcher der Position N320 einer Aminosäuresequenz der SEQ ID NO: 8 entspricht, und eine zu EC 1.14.14.1 gehörende Aktivität aufweist, wobei es sich bei der Aminosäureveränderung um den Austausch des Aminosäurerests, welcher der Position F351 entspricht, gegen Y, T, N, Q, H oder D oder den Austausch des Aminosäurerests, welcher der Position N320 entspricht, gegen W, F, G, P, S oder E handelt.

5. Zusammensetzung zum Entfernen von fluoriertem Methan, dargestellt durch CHₙF₄₋ₙ, wobei es sich bei n um eine ganze Zahl von 0 bis 3 handelt, in einer Probe, wobei die Zusammensetzung den rekombinanten Mikroorganismus nach einem der Ansprüche 1 bis 3, ein Lysat davon oder eine wasserlösliche Fraktion des Lysats und/oder die Variante des bakteriellen P450Cam nach Anspruch 4 und/oder die Variante des bakteriellen P450BM3 nach Anspruch 4 umfasst.

6. Rekombinanter Mikroorganismus nach einem der Ansprüche 1 bis 3 oder Zusammensetzung nach Anspruch 5, wobei es sich bei dem Mikroorganismus um ein grampositives oder gramnegatives Bakterium handelt.

7. Rekombinanter Mikroorganismus oder Zusammensetzung nach Anspruch 6, wobei das gramnegative Bakterium zur Familie *Enterobacteriaceae* gehört.

8. Rekombinanter Mikroorganismus oder Zusammensetzung nach Anspruch 6, wobei das gramnegative Bakterium zur Gattung *Escherichia,* zur Gattung *Salmonella,* zur Gattung *Xanthomonas* oder zur Gattung *Pseudomonas* gehört oder wobei das grampositive Bakterium zur Gattung *Corynebacterium* oder zur Gattung *Bacillus* gehört.

9. Verfahren zum Reduzieren einer Konzentration von fluoriertem Methan in einer Probe, wobei das Verfahren umfasst,
(a) einen rekombinanten Mikroorganismus, umfassend ein exogenes Gen, das ein bakterielles Cytochrom P450 Protein oder eine Variante davon kodiert, wobei es sich bei der Variante um eine P450Cam-Variante handelt, die eine Aminosäureveränderung an einem Aminosäurerest aufweist, welcher der Position F351 einer Aminosäuresequenz der SEQ ID NO: 6 entspricht, und eine zu EC 1.14.15.1 gehörende Aktivität aufweist, oder um eine P450BM3-Variante, die eine Aminosäureveränderung an einem Aminosäurerest aufweist, welcher der Position N320 einer Aminosäuresequenz der SEQ ID NO: 8 entspricht, und eine zu EC 1.14.14.1 gehörende Aktivität aufweist, und/oder
(b) den rekombinanten Mikroorganismus nach einem der Ansprüche 1 bis 3 und 6 bis 8, ein Lysat davon oder eine wasserlösliche Fraktion des Lysats und/oder
(c) die Variante des bakteriellen P450Cam nach Anspruch 4 und/oder
(d) die Variante des bakteriellen P450BM3 nach Anspruch 4 und/oder
(e) die Zusammensetzung nach einem der Ansprüche 5 bis 8 mit der Probe in Kontakt zu bringen, die fluoriertes Methan umfasst, dargestellt durch CHₙF₄₋ₙ, wobei es sich bei n um eine ganze Zahl von 0 bis 3 handelt, um die Konzentration von fluoriertem Methan in der Probe zu reduzieren.

10. Verfahren nach Anspruch 9, wobei das In-Kontakt-Bringen in einem versiegelten Behälter durchgeführt wird.

11. Verfahren nach Anspruch 10, wobei das Verfahren umfasst, den rekombinanten Mikroorganismus nach einem der Ansprüche 1 bis 3, 6 bis 8 und Punkt a) von Anspruch 9 mit der Probe in Kontakt zu bringen, und wobei das In-Kontakt-Bringen in einem versiegelten Behälter unter Bedingungen durchgeführt wird, bei denen der rekombinante Organismus überlebt oder lebensfähig ist.

12. Verfahren nach einem der Ansprüche 9 bis 11, wobei es sich beim In-Kontakt-Bringen um Züchten des rekombinanten Mikroorganismus nach einem der Ansprüche 1 bis 3, 6 bis 8 und Punkt a) von Anspruch 9 in Anwesenheit von fluoriertem Methan handelt.

13. Verfahren nach einem der Ansprüche 9 bis 12, wobei die Probe in einem flüssigen oder gasförmigen Zustand vorliegt, wobei es sich bei der Probe vorzugsweise um Industrieabwasser oder Abgas handelt.

14. Polynucleotid, welches eine Variante des bakteriellen P450Cam kodiert, die eine Aminosäureveränderung an einem Aminosäurerest aufweist, welcher der Position F351 einer Aminosäuresequenz der SEQ ID NO: 6 entspricht, und zu EC 1.14.15.1 gehört oder Polynucleotid, welches eine Variante des bakteriellen P450BM3 kodiert, die eine Aminosäureveränderung an einem Aminosäurerest aufweist, welcher der Position N320 einer Aminosäuresequenz der SEQ ID NO: 8 entspricht, und zu EC 1.14.14.1 gehört, wobei es sich bei der Aminosäureveränderung um den Austausch des Aminosäurerests, welcher der Position F351 entspricht, gegen Y, T, N, Q, H oder D oder den Austausch des Aminosäurerests, welcher der Position N320 entspricht, gegen W, F, G, P, S oder E handelt.

15. Vektor, umfassend das Polynucleotid nach Anspruch 14.

## Revendications

1. Microorganisme recombiné comprenant un gène exogène codant pour un variant de protéine P450 cytochrome bactérienne, le variant étant un variant de P450Cam ayant une altération d'acide aminé au niveau d'un résidu d'acide aminé correspondant à la position F351 d'une séquence d'acides aminés selon la SEQ ID NO : 6 et ayant une activité relevant de EC 1.14.15.1 ou un variant de P450BM3 ayant une altération d'acide aminé au niveau d'un résidu d'acide aminé correspondant à la position N320 d'une séquence d'acides aminés selon la SEQ ID NO : 8 et ayant une activité relevant de EC 1.14.14.1, l'altération d'acide aminé étant un remplacement du résidu d'acide aminé correspondant à la position F351 par Y, T, N, Q, H ou D, ou un remplacement du résidu d'acide aminé correspondant à la position N320 par W, F, G, P, S ou E.

2. Microorganisme recombiné selon la revendication 1, comprenant en outre un gène exogène codant pour une protéine appartenant à EC 1.1.1.49.

3. Microorganisme recombiné selon la revendication 2, dans lequel le gène exogène code pour la glucose-6-phosphate déshydrogénase.

4. Variant de P450Cam bactérien ayant une altération d'acide aminé au niveau d'un résidu d'acide aminé correspondant à la position F351 d'une séquence d'acides aminés selon la SEQ ID NO : 6 et ayant une activité relevant de EC 1.14.15.1 ou variant de P450BM3 bactérien ayant une altération d'acide aminé au niveau d'un résidu d'acide aminé correspondant à la position N320 d'une séquence d'acides aminés selon la SEQ ID NO : 8 et ayant une activité relevant de EC 1.14.14.1, l'altération d'acide aminé étant un remplacement du résidu d'acide aminé correspondant à la position F351 par Y, T, N, Q, H ou D, ou un remplacement du résidu d'acide aminé correspondant à la position N320 par W, F, G, P, S ou E.

5. Composition pour éliminer du méthane fluoré représenté par CHₙF₄₋ₙ, où n est un entier de 0 à 3, dans un échantillon, ladite composition comprenant le microorganisme recombiné selon l'une quelconque des revendications 1 à 3, un lysat de celui-ci ou une fraction hydrosoluble du lysat, et/ou le variant de P450Cam bactérien selon la revendication 4, et/ou le variant de P450BM3 bactérien selon la revendication 4.

6. Microorganisme recombiné selon l'une quelconque des revendications 1 à 3, ou composition selon la revendication 5, le microorganisme étant une bactérie Gram-positive ou Gram-négative.

7. Microorganisme recombiné ou composition selon la revendication 6, la bactérie Gram-négative relevant de la famille des *Enterobacteriaceae.*

8. Microorganisme recombiné ou composition selon la revendication 6, la bactérie Gram-négative appartenant au genre *Escherichia,* au genre *Salmonella,* au genre *Xanthomonas,* ou au genre *Pseudomonas,* ou la bactérie Gram-positive appartenant au genre *Corynebacterium* ou au genre *Bacillus.*

9. Procédé de réduction d'une concentration en méthane fluoré dans un échantillon, le procédé comprenant la mise en contact
a) d'un microorganisme recombiné comprenant un gène exogène codant pour un variant de protéine P450 cytochrome bactérienne, le variant étant un variant de P450Cam ayant une altération d'acide aminé au niveau d'un résidu d'acide aminé correspondant à la position F351 d'une séquence d'acides aminés selon la SEQ ID NO : 6 et ayant une activité relevant de EC 1.14.15.1 ou un variant de P450BM3 ayant une altération d'acide aminé au niveau d'un résidu d'acide aminé correspondant à la position N320 d'une séquence d'acides aminés selon la SEQ ID NO : 8 et ayant une activité relevant de EC 1.14.14.1, et/ou
b) du microorganisme recombiné selon l'une quelconque des revendications 1 à 3 et 6 à 8, d'un lysat de celui-ci ou d'une fraction hydrosoluble du lysat, et/ou
c) du variant de P450Cam bactérien selon la revendication 4, et/ou
d) du variant de P450BM3 bactérien selon la revendication 4, et/ou
e) de la composition selon l'une quelconque des revendications 5 à 8, avec l'échantillon comprenant du méthane fluoré représenté par CHₙF₄₋ₙ, où n est un entier de 0 à 3, pour réduire la concentration de méthane fluoré dans l'échantillon.

10. Procédé selon la revendication 9, dans lequel la mise en contact est effectuée dans un récipient hermétiquement fermé.

11. Procédé selon la revendication 10, le procédé comprenant la mise en contact du microorganisme recombiné selon l'une quelconque des revendications 1 à 3, 6 à 8 et l'article a) de la revendication 9 avec l'échantillon, et la mise en contact étant effectuée dans un récipient hermétiquement fermé dans des conditions dans lesquelles le microorganisme recombiné survit ou est viable.

12. Procédé selon l'une quelconque des revendications 9 à 11, dans lequel la mise en contact consiste à cultiver le microorganisme recombiné selon l'une quelconque des revendications 1 à 3, 6 à 8 et l'article a) de la revendication 9 en présence de méthane fluoré.

13. Procédé selon l'une quelconque des revendications 9 à 12, dans lequel l'échantillon est à l'état liquide ou gazeux, l'échantillon étant de préférence une eau usée ou un effluent gazeux industriel.

14. Polynucléotide codant pour un variant de P450Cam bactérien ayant une altération d'acide aminé au niveau d'un résidu d'acide aminé correspondant à la position F351 d'une séquence d'acides aminés selon la SEQ ID NO : 6 et relevant de EC 1.14.15.1 ou polynucléotide codant pour un variant de P450BM3 bactérien ayant une altération d'acide aminé au niveau d'un résidu d'acide aminé correspondant à la position N320 d'une séquence d'acides aminés selon la SEQ ID NO : 8 et relevant de EC 1.14.14.1, l'altération d'acide aminé étant un remplacement du résidu d'acide aminé correspondant à la position F351 par Y, T, N, Q, H ou D, ou un remplacement du résidu d'acide aminé correspondant à la position N320 par W, F, G, P, S ou E.

15. Vecteur comprenant le polynucléotide selon la revendication 14.


<110> Samsung Electronics Co., Ltd.
<120> BACTERIAL CYTOCHROME P450 PROTEIN VARIANT AND METHOD OF REDUCING CONCENTRATION OF FLUORINATED METHANE IN SAMPLE USING THE SAME
<130> EP109813FZTRpau
<140> not yet assigned
   <141> herewith
<150> 10-2016-0109544
   <151> 2016-08-26
<150> 10-2016-0109543
   <151> 2016-08-26
<150> 10-2016-0075831
   <151> 2016-06-17
<150> 10-2016-0048960
   <151> 2016-04-21
<150> 10-2015-0173293
   <151> 2015-12-07
<160> 56
<170> KopatentIn 2.0
<210> 1
   <211> 1269
   <212> DNA
   <213> P. putida PpG786 strain DSM 7162
<400> 1
   atgaacgcaa acgacaacgt ggtcatcgtc ggtaccggac tggctggcgt tgaggtcgcc 60
   ttcggcctgc gcgccagcgg ctgggaaggc aatatccggt tggtggggga tgcgacggta 120
   attccccatc acctaccacc gctatccaaa gcttacttgg ccggcaaagc cacagcggaa 180
   agcctgtacc tgagaacccc agatgcctat gcagcgcaga acatccaact actcggaggc 240
   acacaggtaa cggctatcaa ccgcgaccga cagcaagtaa tcctatcgga tggccgggca 300
   ctggattacg accggctggt attggctacc ggagggcgtc caagacccct accggtggcc 360
   agtggcgcag ttggaaaggc gaacaacttt cgatacctgc gcacactcga ggacgccgag 420
   tgcattcgcc ggcagctgat tgcggataac cgtctggtgg tgattggtgg cggctacatt 480
   ggccttgaag tggctgccac cgccatcaag gcgaacatgc acgtcaccct gcttgatacg 540
   gcagcccggg ttctggagcg ggttaccgcc ccgccggtat cggcctttta cgagcaccta 600
   caccgcgaag ccggcgttga catacgaacc ggcacgcagg tgtgcgggtt cgagatgtcg 660
   accgaccaac agaaggttac tgccgtcctc tgcgaggacg gcacaaggct gccagcggat 720
   ctggtaatcg ccgggattgg cctgatacca aactgcgagt tggccagtgc ggccggcctg 780
   caggttgata acggcatcgt gatcaacgaa cacatgcaga cctctgatcc cttgatcatg 840
   gccgtcggcg actgtgcccg atttcacagt cagctctatg accgctgggt gcgtatcgaa 900
   tcggtgccca atgccttgga gcaggcacga aagatcgccg ccatcctctg tggcaaggtg 960
   ccacgcgatg aggcggcgcc ctggttctgg tccgatcagt atgagatcgg attgaagatg 1020
   gtcggactgt ccgaagggta cgaccggatc attgtccgcg gctctttggc gcaacccgac 1080
   ttcagcgttt tctacctgca gggagaccgg gtattggcgg tcgatacagt gaaccgtcca 1140
   gtggagttca accagtcaaa acaaataatc acggatcgtt tgccggttga accaaaccta 1200
   ctcggtgacg aaagcgtgcc gttaaaggaa atcatcgccg ccgccaaagc tgaactgagt 1260
   agtgcctaa 1269
<210> 2
   <211> 422
   <212> PRT
   <213> P. putida PpG786 strain DSM 7162
<400> 2
   Met Asn Ala Asn Asp Asn Val Val Ile Val Gly Thr Gly Leu Ala Gly
    1               5                  10                  15
   Val Glu Val Ala Phe Gly Leu Arg Ala Ser Gly Trp Glu Gly Asn Ile
               20                  25                  30
   Arg Leu Val Gly Asp Ala Thr Val Ile Pro His His Leu Pro Pro Leu
           35                  40                  45
   Ser Lys Ala Tyr Leu Ala Gly Lys Ala Thr Ala Glu Ser Leu Tyr Leu
       50                  55                  60
   Arg Thr Pro Asp Ala Tyr Ala Ala Gln Asn Ile Gln Leu Leu Gly Gly
   65                  70                  75                  80
   Thr Gln Val Thr Ala Ile Asn Arg Asp Arg Gln Gln Val Ile Leu Ser
                   85                  90                  95
   Asp Gly Arg Ala Leu Asp Tyr Asp Arg Leu Val Leu Ala Thr Gly Gly
               100                 105                 110
   Arg Pro Arg Pro Leu Pro Val Ala Ser Gly Ala Val Gly Lys Ala Asn
           115                 120                 125
   Asn Phe Arg Tyr Leu Arg Thr Leu Glu Asp Ala Glu Cys Ile Arg Arg
       130                 135                 140
   Gln Leu Ile Ala Asp Asn Arg Leu Val Val Ile Gly Gly Gly Tyr Ile
   145                 150                 155                 160
   Gly Leu Glu Val Ala Ala Thr Ala Ile Lys Ala Asn Met His Val Thr
                   165                 170                 175
   Leu Leu Asp Thr Ala Ala Arg Val Leu Glu Arg Val Thr Ala Pro Pro
               180                 185                 190
   Val Ser Ala Phe Tyr Glu His Leu His Arg Glu Ala Gly Val Asp Ile
           195                 200                 205
   Arg Thr Gly Thr Gln Val Cys Gly Phe Glu Met Ser Thr Asp Gln Gln
       210                 215                 220
   Lys Val Thr Ala Val Leu Cys Glu Asp Gly Thr Arg Leu Pro Ala Asp
   225                 230                 235                 240
   Leu Val Ile Ala Gly Ile Gly Leu Ile Pro Asn Cys Glu Leu Ala Ser
                   245                 250                 255
   Ala Ala Gly Leu Gln Val Asp Asn Gly Ile Val Ile Asn Glu His Met
               260                 265                 270
   Gln Thr Ser Asp Pro Leu Ile Met Ala Val Gly Asp Cys Ala Arg Phe
           275                 280                 285
   His Ser Gln Leu Tyr Asp Arg Trp Val Arg Ile Glu Ser Val Pro Asn
       290                 295                 300
   Ala Leu Glu Gln Ala Arg Lys Ile Ala Ala Ile Leu Cys Gly Lys Val
   305                 310                 315                 320
   Pro Arg Asp Glu Ala Ala Pro Trp Phe Trp Ser Asp Gln Tyr Glu Ile
                   325                 330                 335
   Gly Leu Lys Met Val Gly Leu Ser Glu Gly Tyr Asp Arg Ile Ile Val
               340                 345                 350
   Arg Gly Ser Leu Ala Gln Pro Asp Phe Ser Val Phe Tyr Leu Gln Gly
           355                 360                 365
   Asp Arg Val Leu Ala Val Asp Thr Val Asn Arg Pro Val Glu Phe Asn
       370                 375                 380
   Gln Ser Lys Gln Ile Ile Thr Asp Arg Leu Pro Val Glu Pro Asn Leu
   385                 390                 395                 400
   Leu Gly Asp Glu Ser Val Pro Leu Lys Glu Ile Ile Ala Ala Ala Lys
                   405                 410                 415
   Ala Glu Leu Ser Ser Ala
               420
<210> 3
   <211> 324
   <212> DNA
   <213> P. putida PpG786 strain DSM 7162
<400> 3
   atgtctaaag tagtgtatgt gtcacatgat ggaacgcgtc gcgaactgga tgtggcggat        60
   ggcgtcagcc tgatgcaggc tgcagtctcc aatggtatct acgatattgt cggtgattgt       120
   ggcggcagcg ccagctgtgc cacctgccat gtctatgtga acgaagcgtt cacggacaag       180
   gtgcccgccg ccaacgagcg ggaaatcggc atgctggagt gcgtcacggc cgaactgaag       240
   ccgaacagca ggctctgctg ccagatcatc atgacgcccg agctggatgg catcgtggtc       300
   gatgttcccg ataggcaatg gtaa                                              324
<210> 4
   <211> 107
   <212> PRT
   <213> P. putida PpG786 strain DSM 7162
<400> 4
   Met Ser Lys Val Val Tyr Val Ser His Asp Gly Thr Arg Arg Glu Leu
    1               5                  10                  15
   Asp Val Ala Asp Gly Val Ser Leu Met Gln Ala Ala Val Ser Asn Gly
               20                  25                  30
   Ile Tyr Asp Ile Val Gly Asp Cys Gly Gly Ser Ala Ser Cys Ala Thr
           35                  40                  45
   Cys His Val Tyr Val Asn Glu Ala Phe Thr Asp Lys Val Pro Ala Ala
       50                  55                  60
   Asn Glu Arg Glu Ile Gly Met Leu Glu Cys Val Thr Ala Glu Leu Lys
   65                  70                  75                  80
   Pro Asn Ser Arg Leu Cys Cys Gln Ile Ile Met Thr Pro Glu Leu Asp
                   85                  90                  95
   Gly Ile Val Val Asp Val Pro Asp Arg Gln Trp
               100                 105
<210> 5
   <211> 1248
   <212> DNA
   <213> P. putida PpG786 strain DSM 7162
<400> 5
   atgacgactg aaaccataca aagcaacgcc aatcttgccc ctctgccacc ccatgtgcca      60
   gagcacctgg tattcgactt cgacatgtac aatccgtcga atctgtctgc cggcgtgcag     120
   gaggcctggg cagttctgca agaatcaaac gtaccggatc tggtgtggac tcgctgcaac     180
   ggcggacact ggatcgccac tcgcggccaa ctgatccgtg aggcctatga agattaccgc     240
   cacttttcca gcgagtgccc gttcatccct cgtgaagccg gcgaagccta cgacttcatt     300
   cccacctcga tggatccgcc cgagcagcgc cagtttcgtg cgctggccaa ccaagtggtt     360
   ggcatgccgg tggtggataa gctggagaac cggatccagg agctggcctg ctcgctgatc     420
   gagagcctgc gcccgcaagg acagtgcaac ttcaccgagg actacgccga acccttcccg     480
   atacgcatct tcatgctgct cgcaggtcta ccggaagaag atatcccgca cttgaaatac     540
   ctaacggatc agatgacccg tccggatggc agcatgacct tcgcagaggc caaggaggcg     600
   ctctacgact atctgatacc gatcatcgag caacgcaggc agaagccggg aaccgacgct     660
   atcagcatcg ttgccaacgg ccaggtcaat gggcgaccga tcaccagtga cgaagccaag     720
   aggatgtgtg gcctgttact ggtcggcggc ctggatacgg tggtcaattt cctcagcttc     780
   agcatggagt tcctggccaa aagcccggag catcgccagg agctgatcga gcgtcccgag     840
   cgtattccag ccgcttgcga ggaactactc cggcgcttct cgctggttgc cgatggccgc     900
   atcctcacct ccgattacga gtttcatggc gtgcaactga agaaaggtga ccagatcctg     960
   ctaccgcaga tgctgtctgg cctggatgag cgcgaaaacg cctgcccgat gcacgtcgac    1020
   ttcagtcgcc aaaaggtttc acacaccacc tttggccacg gcagccatct gtgccttggc    1080
   cagcacctgg cccgccggga aatcatcgtc accctcaagg aatggctgac caggattcct    1140
   gacttctcca ttgccccggg tgcccagatt cagcacaaga gcggcatcgt cagcggcgtg    1200
   caggcactcc ctctggtctg ggatccggcg actaccaaag cggtataa                 1248
<210> 6
   <211> 415
   <212> PRT
   <213> P. putida PpG786 strain DSM 7162
<400> 6
   Met Thr Thr Glu Thr Ile Gln Ser Asn Ala Asn Leu Ala Pro Leu Pro
    1               5                  10                  15
   Pro His Val Pro Glu His Leu Val Phe Asp Phe Asp Met Tyr Asn Pro
               20                  25                  30
   Ser Asn Leu Ser Ala Gly Val Gln Glu Ala Trp Ala Val Leu Gln Glu
           35                  40                  45
   Ser Asn Val Pro Asp Leu Val Trp Thr Arg Cys Asn Gly Gly His Trp
       50                  55                  60
   Ile Ala Thr Arg Gly Gln Leu Ile Arg Glu Ala Tyr Glu Asp Tyr Arg
   65                  70                  75                  80
   His Phe Ser Ser Glu Cys Pro Phe Ile Pro Arg Glu Ala Gly Glu Ala
                   85                  90                  95
   Tyr Asp Phe Ile Pro Thr Ser Met Asp Pro Pro Glu Gln Arg Gln Phe
               100                 105                 110
   Arg Ala Leu Ala Asn Gln Val Val Gly Met Pro Val Val Asp Lys Leu
           115                 120                 125
   Glu Asn Arg Ile Gln Glu Leu Ala Cys Ser Leu Ile Glu Ser Leu Arg
       130                 135                 140
   Pro Gln Gly Gln Cys Asn Phe Thr Glu Asp Tyr Ala Glu Pro Phe Pro
   145                 150                 155                 160
   Ile Arg Ile Phe Met Leu Leu Ala Gly Leu Pro Glu Glu Asp Ile Pro
                   165                 170                 175
   His Leu Lys Tyr Leu Thr Asp Gln Met Thr Arg Pro Asp Gly Ser Met
               180                 185                 190
   Thr Phe Ala Glu Ala Lys Glu Ala Leu Tyr Asp Tyr Leu Ile Pro Ile
           195                 200                 205
   Ile Glu Gln Arg Arg Gln Lys Pro Gly Thr Asp Ala Ile Ser Ile Val
       210                 215                 220
   Ala Asn Gly Gln Val Asn Gly Arg Pro Ile Thr Ser Asp Glu Ala Lys
   225                 230                 235                 240
   Arg Met Cys Gly Leu Leu Leu Val Gly Gly Leu Asp Thr Val Val Asn
                   245                 250                 255
   Phe Leu Ser Phe Ser Met Glu Phe Leu Ala Lys Ser Pro Glu His Arg
               260                 265                 270
   Gln Glu Leu Ile Glu Arg Pro Glu Arg Ile Pro Ala Ala Cys Glu Glu
           275                 280                 285
   Leu Leu Arg Arg Phe Ser Leu Val Ala Asp Gly Arg Ile Leu Thr Ser
       290                 295                 300
   Asp Tyr Glu Phe His Gly Val Gln Leu Lys Lys Gly Asp Gln Ile Leu
   305                 310                 315                 320
   Leu Pro Gln Met Leu Ser Gly Leu Asp Glu Arg Glu Asn Ala Cys Pro
                   325                 330                 335
   Met His Val Asp Phe Ser Arg Gln Lys Val Ser His Thr Thr Phe Gly
               340                 345                 350
   His Gly Ser His Leu Cys Leu Gly Gln His Leu Ala Arg Arg Glu Ile
           355                 360                 365
   Ile Val Thr Leu Lys Glu Trp Leu Thr Arg Ile Pro Asp Phe Ser Ile
       370                 375                 380
   Ala Pro Gly Ala Gln Ile Gln His Lys Ser Gly Ile Val Ser Gly Val
   385                 390                 395                 400
   Gln Ala Leu Pro Leu Val Trp Asp Pro Ala Thr Thr Lys Ala Val
                   405                 410                 415
<210> 7
   <211> 3150
   <212> DNA
   <213> Bacillus megaterium (ATCC 14581)
<400> 7
   atgacaatta aagaaatgcc tcagccaaaa acgtttggag agcttaaaaa tttaccgtta      60
   ttaaacacag ataaaccggt tcaagctttg atgaaaattg cggatgaatt aggagaaatc     120
   tttaaattcg aggcgcctgg tcgtgtaacg cgctacttat caagtcagcg tctaattaaa     180
   gaagcatgcg atgaatcacg ctttgataaa aacttaagtc aagcgcttaa atttgtacgt     240
   gattttgcag gagacgggtt atttacaagc tggacgcatg aaaaaaattg gaaaaaagcg     300
   cataatatct tacttccaag cttcagtcag caggcaatga aaggctatca tgcgatgatg     360
   gtcgatatcg ccgtgcagct tgttcaaaag tgggagcgtc taaatgcaga tgagcatatt     420
   gaagtaccgg aagacatgac acgtttaacg cttgatacaa ttggtctttg cggctttaac     480
   tatcgcttta acagctttta ccgagatcag cctcatccat ttattacaag tatggtccgt     540
   gcactggatg aagcaatgaa caagctgcag cgagcaaatc cagacgaccc agcttatgat     600
   gaaaacaagc gccagtttca agaagatatc aaggtgatga acgacctagt agataaaatt     660
   attgcagatc gcaaagcaag cggtgaacaa agcgatgatt tattaacgca tatgctaaac 720
   ggaaaagatc cagaaacggg tgagccgctt gatgacgaga acattcgcta tcaaattatt 780
   acattcttaa ttgcgggaca cgaaacaaca agtggtcttt tatcatttgc gctgtatttc 840
   ttagtgaaaa atccacatgt attacaaaaa gcagcagaag aagcagcacg agttctagta 900
   gatcctgttc caagctacaa acaagtcaaa cagcttaaat atgtcggcat ggtcttaaac 960
   gaagcgctgc gcttatggcc aactgctcct gcgttttccc tatatgcaaa agaagatacg 1020
   gtgcttggag gagaatatcc tttagaaaaa ggcgacgaac taatggttct gattcctcag 1080
   cttcaccgtg ataaaacaat ttggggagac gatgtggaag agttccgtcc agagcgtttt 1140
   gaaaatccaa gtgcgattcc gcagcatgcg tttaaaccgt ttggaaacgg tcagcgtgcg 1200
   tgtatcggtc agcagttcgc tcttcatgaa gcaacgctgg tacttggtat gatgctaaaa 1260
   cactttgact ttgaagatca tacaaactac gagctggata ttaaagaaac tttaacgtta 1320
   aaacctgaag gctttgtggt aaaagcaaaa tcgaaaaaaa ttccgcttgg cggtattcct 1380
   tcacctagca ctgaacagtc tgctaaaaaa gtacgcaaaa aggcagaaaa cgctcataat 1440
   acgccgctgc ttgtgctata cggttcaaat atgggaacag ctgaaggaac ggcgcgtgat 1500
   ttagcagata ttgcaatgag caaaggattt gcaccgcagg tcgcaacgct tgattcacac 1560
   gccggaaatc ttccgcgcga aggagctgta ttaattgtaa cggcgtctta taacggtcat 1620
   ccgcctgata acgcaaagca atttgtcgac tggttagacc aagcgtctgc tgatgaagta 1680
   aaaggcgttc gctactccgt atttggatgc ggcgataaaa actgggctac tacgtatcaa 1740
   aaagtgcctg cttttatcga tgaaacgctt gccgctaaag gggcagaaaa catcgctgac 1800
   cgcggtgaag cagatgcaag cgacgacttt gaaggcacat atgaagaatg gcgtgaacat 1860
   atgtggagtg acgtagcagc ctactttaac ctcgacattg aaaacagtga agataataaa 1920
   tctactcttt cacttcaatt tgtcgacagc gccgcggata tgccgcttgc gaaaatgcac 1980
   ggtgcgtttt caacgaacgt cgtagcaagc aaagaacttc aacagccagg cagtgcacga 2040
   agcacgcgac atcttgaaat tgaacttcca aaagaagctt cttatcaaga aggagatcat 2100
   ttaggtgtta ttcctcgcaa ctatgaagga atagtaaacc gtgtaacagc aaggttcggc 2160
   ctagatgcat cacagcaaat ccgtctggaa gcagaagaag aaaaattagc tcatttgcca 2220
   ctcgctaaaa cagtatccgt agaagagctt ctgcaatacg tggagcttca agatcctgtt 2280
   acgcgcacgc agcttcgcgc aatggctgct aaaacggtct gcccgccgca taaagtagag 2340
   cttgaagcct tgcttgaaaa gcaagcctac aaagaacaag tgctggcaaa acgtttaaca 2400
   atgcttgaac tgcttgaaaa atacccggcg tgtgaaatga aattcagcga atttatcgcc 2460
   cttctgccaa gcatacgccc gcgctattac tcgatttctt catcacctcg tgtcgatgaa 2520
   aaacaagcaa gcatcacggt cagcgttgtc tcaggagaag cgtggagcgg atatggagaa 2580
   tataaaggaa ttgcgtcgaa ctatcttgcc gagctgcaag aaggagatac gattacgtgc    2640
   tttatttcca caccgcagtc agaatttacg ctgccaaaag accctgaaac gccgcttatc    2700
   atggtcggac cgggaacagg cgtcgcgccg tttagaggct ttgtgcaggc gcgcaaacag    2760
   ctaaaagaac aaggacagtc acttggagaa gcacatttat acttcggctg ccgttcacct    2820
   catgaagact atctgtatca agaagagctt gaaaacgccc aaagcgaagg catcattacg    2880
   cttcataccg ctttttctcg catgccaaat cagccgaaaa catacgttca gcacgtaatg    2940
   gaacaagacg gcaagaaatt gattgaactt cttgatcaag gagcgcactt ctatatttgc    3000
   ggagacggaa gccaaatggc acctgccgtt gaagcaacgc ttatgaaaag ctatgctgac    3060
   gttcaccaag tgagtgaagc agacgctcgc ttatggctgc agcagctaga agaaaaaggc    3120
   cgatacgcaa aagacgtgtg ggctgggtaa                                      3150
<210> 8
   <211> 1049
   <212> PRT
   <213> Bacillus megaterium (ATCC 14581)
<400> 8
<210> 9
   <211> 1476
   <212> DNA
   <213> E. coli K12 (MG1655)
<400> 9
   atggcggtaa cgcaaacagc ccaggcctgt gacctggtca ttttcggcgc gaaaggcgac     60
   cttgcgcgtc gtaaattgct gccttccctg tatcaactgg aaaaagccgg tcagctcaac    120
   ccggacaccc ggattatcgg cgtagggcgt gctgactggg ataaagcggc atataccaaa    180
   gttgtccgcg aggcgctcga aactttcatg aaagaaacca ttgatgaagg tttatgggac    240
   accctgagtg cacgtctgga tttttgtaat ctcgatgtca atgacactgc tgcattcagc    300
   cgtctcggcg cgatgctgga tcaaaaaaat cgtatcacca ttaactactt tgccatgccg    360
   cccagcactt ttggcgcaat ttgcaaaggg cttggcgagg caaaactgaa tgctaaaccg    420
   gcacgcgtag tcatggagaa accgctgggg acgtcgctgg cgacctcgca ggaaatcaat    480
   gatcaggttg gcgaatactt cgaggagtgc caggtttacc gtatcgacca ctatcttggt    540
   aaagaaacgg tgctgaacct gttggcgctg cgttttgcta actccctgtt tgtgaataac    600
   tgggacaatc gcaccattga tcatgttgag attaccgtgg cagaagaagt ggggatcgaa    660
   gggcgctggg gctattttga taaagccggt cagatgcgcg acatgatcca gaaccacctg    720
   ctgcaaattc tttgcatgat tgcgatgtct ccgccgtctg acctgagcgc agacagcatc    780
   cgcgatgaaa aagtgaaagt actgaagtct ctgcgccgca tcgaccgctc caacgtacgc    840
   gaaaaaaccg tacgcgggca atatactgcg ggcttcgccc agggcaaaaa agtgccggga    900
   tatctggaag aagagggcgc gaacaagagc agcaatacag aaactttcgt ggcgatccgc    960
   gtcgacattg ataactggcg ctgggccggt gtgccattct acctgcgtac tggtaaacgt   1020
   ctgccgacca aatgttctga agtcgtggtc tatttcaaaa cacctgaact gaatctgttt   1080
   aaagaatcgt ggcaggatct gccgcagaat aaactgacta tccgtctgca acctgatgaa   1140
   ggcgtggata tccaggtact gaataaagtt cctggccttg accacaaaca taacctgcaa   1200
   atcaccaagc tggatctgag ctattcagaa acctttaatc agacgcatct ggcggatgcc   1260
   tatgaacgtt tgctgctgga aaccatgcgt ggtattcagg cactgtttgt acgtcgcgac   1320
   gaagtggaag aagcctggaa atgggtagac tccattactg aggcgtgggc gatggacaat   1380
   gatgcgccga aaccgtatca ggccggaacc tggggacccg ttgcctcggt ggcgatgatt   1440
   acccgtgatg gtcgttcctg gaatgagttt gagtaa                              1476
<210> 10
   <211> 491
   <212> PRT
   <213> E. coli K12 (MG1655)
<400> 10
   Met Ala Val Thr Gln Thr Ala Gln Ala Cys Asp Leu Val Ile Phe Gly
   1 5 10 15
   Ala Lys Gly Asp Leu Ala Arg Arg Lys Leu Leu Pro Ser Leu Tyr Gln
   20 25 30
   Leu Glu Lys Ala Gly Gln Leu Asn Pro Asp Thr Arg Ile Ile Gly Val
   35 40 45
   Gly Arg Ala Asp Trp Asp Lys Ala Ala Tyr Thr Lys Val Val Arg Glu
   50 55 60
   Ala Leu Glu Thr Phe Met Lys Glu Thr Ile Asp Glu Gly Leu Trp Asp
   65 70 75 80
   Thr Leu Ser Ala Arg Leu Asp Phe Cys Asn Leu Asp Val Asn Asp Thr
   85 90 95
   Ala Ala Phe Ser Arg Leu Gly Ala Met Leu Asp Gln Lys Asn Arg Ile
   100 105 110
   Thr Ile Asn Tyr Phe Ala Met Pro Pro Ser Thr Phe Gly Ala Ile Cys
   115 120 125
   Lys Gly Leu Gly Glu Ala Lys Leu Asn Ala Lys Pro Ala Arg Val Val
   130 135 140
   Met Glu Lys Pro Leu Gly Thr Ser Leu Ala Thr Ser Gln Glu Ile Asn
   145 150 155 160
   Asp Gln Val Gly Glu Tyr Phe Glu Glu Cys Gln Val Tyr Arg Ile Asp
   165 170 175
   His Tyr Leu Gly Lys Glu Thr Val Leu Asn Leu Leu Ala Leu Arg Phe
   180 185 190
   Ala Asn Ser Leu Phe Val Asn Asn Trp Asp Asn Arg Thr Ile Asp His
   195 200 205
   Val Glu Ile Thr Val Ala Glu Glu Val Gly Ile Glu Gly Arg Trp Gly
   210 215 220
   Tyr Phe Asp Lys Ala Gly Gln Met Arg Asp Met Ile Gln Asn His Leu
   225 230 235 240
   Leu Gln Ile Leu Cys Met Ile Ala Met Ser Pro Pro Ser Asp Leu Ser
   245 250 255
   Ala Asp Ser Ile Arg Asp Glu Lys Val Lys Val Leu Lys Ser Leu Arg
   260 265 270
   Arg Ile Asp Arg Ser Asn Val Arg Glu Lys Thr Val Arg Gly Gln Tyr
   275 280 285
   Thr Ala Gly Phe Ala Gln Gly Lys Lys Val Pro Gly Tyr Leu Glu Glu
   290 295 300
   Glu Gly Ala Asn Lys Ser Ser Asn Thr Glu Thr Phe Val Ala Ile Arg
   305 310 315 320
   Val Asp Ile Asp Asn Trp Arg Trp Ala Gly Val Pro Phe Tyr Leu Arg
   325 330 335
   Thr Gly Lys Arg Leu Pro Thr Lys Cys Ser Glu Val Val Val Tyr Phe
   340 345 350
   Lys Thr Pro Glu Leu Asn Leu Phe Lys Glu Ser Trp Gln Asp Leu Pro
   355 360 365
   Gln Asn Lys Leu Thr Ile Arg Leu Gln Pro Asp Glu Gly Val Asp Ile
   370 375 380
   Gln Val Leu Asn Lys Val Pro Gly Leu Asp His Lys His Asn Leu Gln
   385 390 395 400
   Ile Thr Lys Leu Asp Leu Ser Tyr Ser Glu Thr Phe Asn Gln Thr His
   405 410 415
   Leu Ala Asp Ala Tyr Glu Arg Leu Leu Leu Glu Thr Met Arg Gly Ile
   420 425 430
   Gln Ala Leu Phe Val Arg Arg Asp Glu Val Glu Glu Ala Trp Lys Trp
   435 440 445
   Val Asp Ser Ile Thr Glu Ala Trp Ala Met Asp Asn Asp Ala Pro Lys
   450 455 460
   Pro Tyr Gln Ala Gly Thr Trp Gly Pro Val Ala Ser Val Ala Met Ile
   465 470 475 480
   Thr Arg Asp Gly Arg Ser Trp Asn Glu Phe Glu
   485 490
<210> 11
   <211> 37
   <212> DNA
   <213> Artificial Sequence
<220>
   <223> Primer: camA_F
<400> 11
   taagaaggag atatacatat gaacgcaaac gacaacg 37
<210> 12
   <211> 42
   <212> DNA
   <213> Artificial Sequence
<220>
   <223> Primer: camA_R
<400> 12
   catgaattct gtttcctgtg tgattaggca ctactcagtt ca 42
<210> 13
   <211> 42
   <212> DNA
   <213> Artificial Sequence
<220>
   <223> Primer: camB_F
<400> 13
   taatcacaca ggaaacagaa ttcatgtcta aagtagtgta tg 42
<210> 14
   <211> 40
   <212> DNA
   <213> Artificial Sequence
<220>
   <223> Primer: camB_R
<400> 14
   ggtttcttta ccagactcga ttaccattgc ctatcgggaa 40
<210> 15
   <211> 36
   <212> DNA
   <213> Artificial Sequence
<220>
   <223> Primer: camC_F
<400> 15
   aagaaggaga tataccatga cgactgaaac cataca 36
<210> 16
   <211> 40
   <212> DNA
   <213> Artificial Sequence
<220>
   <223> Primer: camC_R
<400> 16
   gcattatgcg gccgcaagct ttataccgct ttggtagtcg 40
<210> 17
   <211> 37
   <212> DNA
   <213> Artificial Sequence
<220>
   <223> Primer: P450bm3_F
<400> 17
   aagaaggaga tataccatga caattaaaga aatgcct 37
<210> 18
   <211> 40
   <212> DNA
   <213> Artificial Sequence
<220>
   <223> Primer: P450bm3_R
<400> 18
   gtggtggtgg tggtgctcga ttacccagcc cacacgtctt 40
<210> 19
   <211> 38
   <212> DNA
   <213> Artificial Sequence
<220>
   <223> Primer: Zwf_F
<400> 19
   ttaagaagga gatataccat ggcggtaacg caaacagc 38
<210> 20
   <211> 39
   <212> DNA
   <213> Artificial Sequence
<220>
   <223> Primer: Zwf_R
<400> 20
   tcgacctgca ggcgcgccgt tactcaaact cattccagg 39
<210> 21
   <211> 39
   <212> DNA
   <213> Artificial Sequence
<220>
   <223> Forward primer for F351Y
<400> 21
   aaggtttcac acaccaccta tggccacggc agccatctg 39
<210> 22
   <211> 39
   <212> DNA
   <213> Artificial Sequence
<220>
   <223> Reverse primer for F351Y
<400> 22
   cagatggctg ccgtggccat aggtggtgtg tgaaacctt 39
<210> 23
   <211> 39
   <212> DNA
   <213> Artificial Sequence
<220>
   <223> Forward primer for F351T
<400> 23
   aaggtttcac acaccaccac cggccacggc agccatctg 39
<210> 24
   <211> 39
   <212> DNA
   <213> Artificial Sequence
<220>
   <223> Reverse primer for F351T
<400> 24
   cagatggctg ccgtggccgg tggtggtgtg tgaaacctt 39
<210> 25
   <211> 39
   <212> DNA
   <213> Artificial Sequence
<220>
   <223> Forward primer for F351N
<400> 25
   aaggtttcac acaccaccaa cggccacggc agccatctg 39
<210> 26
   <211> 39
   <212> DNA
   <213> Artificial Sequence
<220>
   <223> Reverse primer for F351N
<400> 26
   cagatggctg ccgtggccgt tggtggtgtg tgaaacctt 39
<210> 27
   <211> 39
   <212> DNA
   <213> Artificial Sequence
<220>
   <223> Forward primer for F351Q
<400> 27
   aaggtttcac acaccaccca gggccacggc agccatctg 39
<210> 28
   <211> 39
   <212> DNA
   <213> Artificial Sequence
<220>
   <223> Reverse primer for F351Q
<400> 28
   cagatggctg ccgtggccct gggtggtgtg tgaaacctt 39
<210> 29
   <211> 39
   <212> DNA
   <213> Artificial Sequence
<220>
   <223> Forward primer for F351H
<400> 29
   aaggtttcac acaccaccca tggccacggc agccatctg 39
<210> 30
   <211> 39
   <212> DNA
   <213> Artificial Sequence
<220>
   <223> Reverse primer for F351H
<400> 30
   cagatggctg ccgtggccat gggtggtgtg tgaaacctt 39
<210> 31
   <211> 39
   <212> DNA
   <213> Artificial Sequence
<220>
   <223> Forward primer for F351D
<400> 31
   aaggtttcac acaccaccga tggccacggc agccatctg 39
<210> 32
   <211> 39
   <212> DNA
   <213> Artificial Sequence
<220>
   <223> Reverse primer for F351D
<400> 32
   cagatggctg ccgtggccat cggtggtgtg tgaaacctt 39
<210> 33
   <211> 39
   <212> DNA
   <213> Artificial Sequence
<220>
   <223> Forward primer for N320W
<400> 33
   tatgtcggca tggtcttatg ggaagcgctg cgcttatgg 39
<210> 34
   <211> 39
   <212> DNA
   <213> Artificial Sequence
<220>
   <223> Reverse primer for N320W
<400> 34
   ccataagcgc agcgcttccc ataagaccat gccgacata 39
<210> 35
   <211> 39
   <212> DNA
   <213> Artificial Sequence
<220>
   <223> Forward primer for N320F
<400> 35
   tatgtcggca tggtcttatt tgaagcgctg cgcttatgg 39
<210> 36
   <211> 39
   <212> DNA
   <213> Artificial Sequence
<220>
   <223> Reverse primer for N320F
<400> 36
   ccataagcgc agcgcttcaa ataagaccat gccgacata 39
<210> 37
   <211> 39
   <212> DNA
   <213> Artificial Sequence
<220>
   <223> Forward primer for N320G
<400> 37
   tatgtcggca tggtcttagg cgaagcgctg cgcttatgg 39
<210> 38
   <211> 39
   <212> DNA
   <213> Artificial Sequence
<220>
   <223> Reverse primer for N320G
<400> 38
   ccataagcgc agcgcttcgc ctaagaccat gccgacata 39
<210> 39
   <211> 39
   <212> DNA
   <213> Artificial Sequence
<220>
   <223> Forward primer for N320P
<400> 39
   tatgtcggca tggtcttacc ggaagcgctg cgcttatgg 39
<210> 40
   <211> 39
   <212> DNA
   <213> Artificial Sequence
<220>
   <223> Reverse primer for N320P
<400> 40
   ccataagcgc agcgcttccg gtaagaccat gccgacata 39
<210> 41
   <211> 39
   <212> DNA
   <213> Artificial Sequence
<220>
   <223> Forward primer for N320S
<400> 41
   tatgtcggca tggtcttaag cgaagcgctg cgcttatgg 39
<210> 42
   <211> 39
   <212> DNA
   <213> Artificial Sequence
<220>
   <223> Reverse primer for N320S
<400> 42
   ccataagcgc agcgcttcgc ttaagaccat gccgacata 39
<210> 43
   <211> 39
   <212> DNA
   <213> Artificial Sequence
<220>
   <223> Forward primer for N320E
<400> 43
   tatgtcggca tggtcttaga agaagcgctg cgcttatgg 39
<210> 44
   <211> 39
   <212> DNA
   <213> Artificial Sequence
<220>
   <223> Reverse primer for N320E
<400> 44
   ccataagcgc agcgcttctt ctaagaccat gccgacata 39
<210> 45
   <211> 3150
   <212> DNA
   <213> Artificial Sequence
<220>
   <223> P450BM3 N320W gene
<400> 45
   atgacaatta aagaaatgcc tcagccaaaa acgtttggag agcttaaaaa tttaccgtta      60
   ttaaacacag ataaaccggt tcaagctttg atgaaaattg cggatgaatt aggagaaatc     120
   tttaaattcg aggcgcctgg tcgtgtaacg cgctacttat caagtcagcg tctaattaaa     180
   gaagcatgcg atgaatcacg ctttgataaa aacttaagtc aagcgcttaa atttgtacgt     240
   gattttgcag gagacgggtt atttacaagc tggacgcatg aaaaaaattg gaaaaaagcg     300
   cataatatct tacttccaag cttcagtcag caggcaatga aaggctatca tgcgatgatg     360
   gtcgatatcg ccgtgcagct tgttcaaaag tgggagcgtc taaatgcaga tgagcatatt     420
   gaagtaccgg aagacatgac acgtttaacg cttgatacaa ttggtctttg cggctttaac     480
   tatcgcttta acagctttta ccgagatcag cctcatccat ttattacaag tatggtccgt     540
   gcactggatg aagcaatgaa caagctgcag cgagcaaatc cagacgaccc agcttatgat     600
   gaaaacaagc gccagtttca agaagatatc aaggtgatga acgacctagt agataaaatt     660
   attgcagatc gcaaagcaag cggtgaacaa agcgatgatt tattaacgca tatgctaaac     720
   ggaaaagatc cagaaacggg tgagccgctt gatgacgaga acattcgcta tcaaattatt     780
   acattcttaa ttgcgggaca cgaaacaaca agtggtcttt tatcatttgc gctgtatttc     840
   ttagtgaaaa atccacatgt attacaaaaa gcagcagaag aagcagcacg agttctagta     900
   gatcctgttc caagctacaa acaagtcaaa cagcttaaat atgtcggcat ggtcttatgg     960
   gaagcgctgc gcttatggcc aactgctcct gcgttttccc tatatgcaaa agaagatacg    1020
   gtgcttggag gagaatatcc tttagaaaaa ggcgacgaac taatggttct gattcctcag    1080
   cttcaccgtg ataaaacaat ttggggagac gatgtggaag agttccgtcc agagcgtttt    1140
   gaaaatccaa gtgcgattcc gcagcatgcg tttaaaccgt ttggaaacgg tcagcgtgcg    1200
   tgtatcggtc agcagttcgc tcttcatgaa gcaacgctgg tacttggtat gatgctaaaa    1260
   cactttgact ttgaagatca tacaaactac gagctggata ttaaagaaac tttaacgtta    1320
   aaacctgaag gctttgtggt aaaagcaaaa tcgaaaaaaa ttccgcttgg cggtattcct    1380
   tcacctagca ctgaacagtc tgctaaaaaa gtacgcaaaa aggcagaaaa cgctcataat 1440
   acgccgctgc ttgtgctata cggttcaaat atgggaacag ctgaaggaac ggcgcgtgat 1500
   ttagcagata ttgcaatgag caaaggattt gcaccgcagg tcgcaacgct tgattcacac 1560
   gccggaaatc ttccgcgcga aggagctgta ttaattgtaa cggcgtctta taacggtcat 1620
   ccgcctgata acgcaaagca atttgtcgac tggttagacc aagcgtctgc tgatgaagta 1680
   aaaggcgttc gctactccgt atttggatgc ggcgataaaa actgggctac tacgtatcaa 1740
   aaagtgcctg cttttatcga tgaaacgctt gccgctaaag gggcagaaaa catcgctgac 1800
   cgcggtgaag cagatgcaag cgacgacttt gaaggcacat atgaagaatg gcgtgaacat 1860
   atgtggagtg acgtagcagc ctactttaac ctcgacattg aaaacagtga agataataaa 1920
   tctactcttt cacttcaatt tgtcgacagc gccgcggata tgccgcttgc gaaaatgcac 1980
   ggtgcgtttt caacgaacgt cgtagcaagc aaagaacttc aacagccagg cagtgcacga 2040
   agcacgcgac atcttgaaat tgaacttcca aaagaagctt cttatcaaga aggagatcat 2100
   ttaggtgtta ttcctcgcaa ctatgaagga atagtaaacc gtgtaacagc aaggttcggc 2160
   ctagatgcat cacagcaaat ccgtctggaa gcagaagaag aaaaattagc tcatttgcca 2220
   ctcgctaaaa cagtatccgt agaagagctt ctgcaatacg tggagcttca agatcctgtt 2280
   acgcgcacgc agcttcgcgc aatggctgct aaaacggtct gcccgccgca taaagtagag 2340
   cttgaagcct tgcttgaaaa gcaagcctac aaagaacaag tgctggcaaa acgtttaaca 2400
   atgcttgaac tgcttgaaaa atacccggcg tgtgaaatga aattcagcga atttatcgcc 2460
   cttctgccaa gcatacgccc gcgctattac tcgatttctt catcacctcg tgtcgatgaa 2520
   aaacaagcaa gcatcacggt cagcgttgtc tcaggagaag cgtggagcgg atatggagaa 2580
   tataaaggaa ttgcgtcgaa ctatcttgcc gagctgcaag aaggagatac gattacgtgc 2640
   tttatttcca caccgcagtc agaatttacg ctgccaaaag accctgaaac gccgcttatc 2700
   atggtcggac cgggaacagg cgtcgcgccg tttagaggct ttgtgcaggc gcgcaaacag 2760
   ctaaaagaac aaggacagtc acttggagaa gcacatttat acttcggctg ccgttcacct 2820
   catgaagact atctgtatca agaagagctt gaaaacgccc aaagcgaagg catcattacg 2880
   cttcataccg ctttttctcg catgccaaat cagccgaaaa catacgttca gcacgtaatg 2940
   gaacaagacg gcaagaaatt gattgaactt cttgatcaag gagcgcactt ctatatttgc 3000
   ggagacggaa gccaaatggc acctgccgtt gaagcaacgc ttatgaaaag ctatgctgac 3060
   gttcaccaag tgagtgaagc agacgctcgc ttatggctgc agcagctaga agaaaaaggc 3120
   cgatacgcaa aagacgtgtg ggctgggtaa 3150
<210> 46
   <211> 3150
   <212> DNA
   <213> Artificial Sequence
<220>
   <223> P450BM3 N320F gene
<400> 46
   atgacaatta aagaaatgcc tcagccaaaa acgtttggag agcttaaaaa tttaccgtta        60
   ttaaacacag ataaaccggt tcaagctttg atgaaaattg cggatgaatt aggagaaatc       120
   tttaaattcg aggcgcctgg tcgtgtaacg cgctacttat caagtcagcg tctaattaaa       180
   gaagcatgcg atgaatcacg ctttgataaa aacttaagtc aagcgcttaa atttgtacgt       240
   gattttgcag gagacgggtt atttacaagc tggacgcatg aaaaaaattg gaaaaaagcg       300
   cataatatct tacttccaag cttcagtcag caggcaatga aaggctatca tgcgatgatg       360
   gtcgatatcg ccgtgcagct tgttcaaaag tgggagcgtc taaatgcaga tgagcatatt       420
   gaagtaccgg aagacatgac acgtttaacg cttgatacaa ttggtctttg cggctttaac       480
   tatcgcttta acagctttta ccgagatcag cctcatccat ttattacaag tatggtccgt       540
   gcactggatg aagcaatgaa caagctgcag cgagcaaatc cagacgaccc agcttatgat       600
   gaaaacaagc gccagtttca agaagatatc aaggtgatga acgacctagt agataaaatt       660
   attgcagatc gcaaagcaag cggtgaacaa agcgatgatt tattaacgca tatgctaaac       720
   ggaaaagatc cagaaacggg tgagccgctt gatgacgaga acattcgcta tcaaattatt       780
   acattcttaa ttgcgggaca cgaaacaaca agtggtcttt tatcatttgc gctgtatttc       840
   ttagtgaaaa atccacatgt attacaaaaa gcagcagaag aagcagcacg agttctagta       900
   gatcctgttc caagctacaa acaagtcaaa cagcttaaat atgtcggcat ggtcttattt       960
   gaagcgctgc gcttatggcc aactgctcct gcgttttccc tatatgcaaa agaagatacg      1020
   gtgcttggag gagaatatcc tttagaaaaa ggcgacgaac taatggttct gattcctcag      1080
   cttcaccgtg ataaaacaat ttggggagac gatgtggaag agttccgtcc agagcgtttt      1140
   gaaaatccaa gtgcgattcc gcagcatgcg tttaaaccgt ttggaaacgg tcagcgtgcg      1200
   tgtatcggtc agcagttcgc tcttcatgaa gcaacgctgg tacttggtat gatgctaaaa      1260
   cactttgact ttgaagatca tacaaactac gagctggata ttaaagaaac tttaacgtta      1320
   aaacctgaag gctttgtggt aaaagcaaaa tcgaaaaaaa ttccgcttgg cggtattcct      1380
   tcacctagca ctgaacagtc tgctaaaaaa gtacgcaaaa aggcagaaaa cgctcataat      1440
   acgccgctgc ttgtgctata cggttcaaat atgggaacag ctgaaggaac ggcgcgtgat      1500
   ttagcagata ttgcaatgag caaaggattt gcaccgcagg tcgcaacgct tgattcacac      1560
   gccggaaatc ttccgcgcga aggagctgta ttaattgtaa cggcgtctta taacggtcat      1620
   ccgcctgata acgcaaagca atttgtcgac tggttagacc aagcgtctgc tgatgaagta      1680
   aaaggcgttc gctactccgt atttggatgc ggcgataaaa actgggctac tacgtatcaa        1740
   aaagtgcctg cttttatcga tgaaacgctt gccgctaaag gggcagaaaa catcgctgac        1800
   cgcggtgaag cagatgcaag cgacgacttt gaaggcacat atgaagaatg gcgtgaacat        1860
   atgtggagtg acgtagcagc ctactttaac ctcgacattg aaaacagtga agataataaa        1920
   tctactcttt cacttcaatt tgtcgacagc gccgcggata tgccgcttgc gaaaatgcac        1980
   ggtgcgtttt caacgaacgt cgtagcaagc aaagaacttc aacagccagg cagtgcacga        2040
   agcacgcgac atcttgaaat tgaacttcca aaagaagctt cttatcaaga aggagatcat        2100
   ttaggtgtta ttcctcgcaa ctatgaagga atagtaaacc gtgtaacagc aaggttcggc        2160
   ctagatgcat cacagcaaat ccgtctggaa gcagaagaag aaaaattagc tcatttgcca        2220
   ctcgctaaaa cagtatccgt agaagagctt ctgcaatacg tggagcttca agatcctgtt        2280
   acgcgcacgc agcttcgcgc aatggctgct aaaacggtct gcccgccgca taaagtagag        2340
   cttgaagcct tgcttgaaaa gcaagcctac aaagaacaag tgctggcaaa acgtttaaca        2400
   atgcttgaac tgcttgaaaa atacccggcg tgtgaaatga aattcagcga atttatcgcc        2460
   cttctgccaa gcatacgccc gcgctattac tcgatttctt catcacctcg tgtcgatgaa        2520
   aaacaagcaa gcatcacggt cagcgttgtc tcaggagaag cgtggagcgg atatggagaa        2580
   tataaaggaa ttgcgtcgaa ctatcttgcc gagctgcaag aaggagatac gattacgtgc        2640
   tttatttcca caccgcagtc agaatttacg ctgccaaaag accctgaaac gccgcttatc        2700
   atggtcggac cgggaacagg cgtcgcgccg tttagaggct ttgtgcaggc gcgcaaacag        2760
   ctaaaagaac aaggacagtc acttggagaa gcacatttat acttcggctg ccgttcacct        2820
   catgaagact atctgtatca agaagagctt gaaaacgccc aaagcgaagg catcattacg        2880
   cttcataccg ctttttctcg catgccaaat cagccgaaaa catacgttca gcacgtaatg        2940
   gaacaagacg gcaagaaatt gattgaactt cttgatcaag gagcgcactt ctatatttgc        3000
   ggagacggaa gccaaatggc acctgccgtt gaagcaacgc ttatgaaaag ctatgctgac        3060
   gttcaccaag tgagtgaagc agacgctcgc ttatggctgc agcagctaga agaaaaaggc        3120
   cgatacgcaa aagacgtgtg ggctgggtaa                                         3150
<210> 47
   <211> 3150
   <212> DNA
   <213> Artificial Sequence
<220>
   <223> P450BM3 N320G
<400> 47
   atgacaatta aagaaatgcc tcagccaaaa acgtttggag agcttaaaaa tttaccgtta 60
   ttaaacacag ataaaccggt tcaagctttg atgaaaattg cggatgaatt aggagaaatc 120
   tttaaattcg aggcgcctgg tcgtgtaacg cgctacttat caagtcagcg tctaattaaa 180
   gaagcatgcg atgaatcacg ctttgataaa aacttaagtc aagcgcttaa atttgtacgt 240
   gattttgcag gagacgggtt atttacaagc tggacgcatg aaaaaaattg gaaaaaagcg 300
   cataatatct tacttccaag cttcagtcag caggcaatga aaggctatca tgcgatgatg 360
   gtcgatatcg ccgtgcagct tgttcaaaag tgggagcgtc taaatgcaga tgagcatatt 420
   gaagtaccgg aagacatgac acgtttaacg cttgatacaa ttggtctttg cggctttaac 480
   tatcgcttta acagctttta ccgagatcag cctcatccat ttattacaag tatggtccgt 540
   gcactggatg aagcaatgaa caagctgcag cgagcaaatc cagacgaccc agcttatgat 600
   gaaaacaagc gccagtttca agaagatatc aaggtgatga acgacctagt agataaaatt 660
   attgcagatc gcaaagcaag cggtgaacaa agcgatgatt tattaacgca tatgctaaac 720
   ggaaaagatc cagaaacggg tgagccgctt gatgacgaga acattcgcta tcaaattatt 780
   acattcttaa ttgcgggaca cgaaacaaca agtggtcttt tatcatttgc gctgtatttc 840
   ttagtgaaaa atccacatgt attacaaaaa gcagcagaag aagcagcacg agttctagta 900
   gatcctgttc caagctacaa acaagtcaaa cagcttaaat atgtcggcat ggtcttaggc 960
   gaagcgctgc gcttatggcc aactgctcct gcgttttccc tatatgcaaa agaagatacg 1020
   gtgcttggag gagaatatcc tttagaaaaa ggcgacgaac taatggttct gattcctcag 1080
   cttcaccgtg ataaaacaat ttggggagac gatgtggaag agttccgtcc agagcgtttt 1140
   gaaaatccaa gtgcgattcc gcagcatgcg tttaaaccgt ttggaaacgg tcagcgtgcg 1200
   tgtatcggtc agcagttcgc tcttcatgaa gcaacgctgg tacttggtat gatgctaaaa 1260
   cactttgact ttgaagatca tacaaactac gagctggata ttaaagaaac tttaacgtta 1320
   aaacctgaag gctttgtggt aaaagcaaaa tcgaaaaaaa ttccgcttgg cggtattcct 1380
   tcacctagca ctgaacagtc tgctaaaaaa gtacgcaaaa aggcagaaaa cgctcataat 1440
   acgccgctgc ttgtgctata cggttcaaat atgggaacag ctgaaggaac ggcgcgtgat 1500
   ttagcagata ttgcaatgag caaaggattt gcaccgcagg tcgcaacgct tgattcacac 1560
   gccggaaatc ttccgcgcga aggagctgta ttaattgtaa cggcgtctta taacggtcat 1620
   ccgcctgata acgcaaagca atttgtcgac tggttagacc aagcgtctgc tgatgaagta 1680
   aaaggcgttc gctactccgt atttggatgc ggcgataaaa actgggctac tacgtatcaa 1740
   aaagtgcctg cttttatcga tgaaacgctt gccgctaaag gggcagaaaa catcgctgac 1800
   cgcggtgaag cagatgcaag cgacgacttt gaaggcacat atgaagaatg gcgtgaacat 1860
   atgtggagtg acgtagcagc ctactttaac ctcgacattg aaaacagtga agataataaa 1920
   tctactcttt cacttcaatt tgtcgacagc gccgcggata tgccgcttgc gaaaatgcac    1980
   ggtgcgtttt caacgaacgt cgtagcaagc aaagaacttc aacagccagg cagtgcacga    2040
   agcacgcgac atcttgaaat tgaacttcca aaagaagctt cttatcaaga aggagatcat    2100
   ttaggtgtta ttcctcgcaa ctatgaagga atagtaaacc gtgtaacagc aaggttcggc    2160
   ctagatgcat cacagcaaat ccgtctggaa gcagaagaag aaaaattagc tcatttgcca    2220
   ctcgctaaaa cagtatccgt agaagagctt ctgcaatacg tggagcttca agatcctgtt    2280
   acgcgcacgc agcttcgcgc aatggctgct aaaacggtct gcccgccgca taaagtagag    2340
   cttgaagcct tgcttgaaaa gcaagcctac aaagaacaag tgctggcaaa acgtttaaca    2400
   atgcttgaac tgcttgaaaa atacccggcg tgtgaaatga aattcagcga atttatcgcc    2460
   cttctgccaa gcatacgccc gcgctattac tcgatttctt catcacctcg tgtcgatgaa    2520
   aaacaagcaa gcatcacggt cagcgttgtc tcaggagaag cgtggagcgg atatggagaa    2580
   tataaaggaa ttgcgtcgaa ctatcttgcc gagctgcaag aaggagatac gattacgtgc    2640
   tttatttcca caccgcagtc agaatttacg ctgccaaaag accctgaaac gccgcttatc    2700
   atggtcggac cgggaacagg cgtcgcgccg tttagaggct ttgtgcaggc gcgcaaacag    2760
   ctaaaagaac aaggacagtc acttggagaa gcacatttat acttcggctg ccgttcacct    2820
   catgaagact atctgtatca agaagagctt gaaaacgccc aaagcgaagg catcattacg    2880
   cttcataccg ctttttctcg catgccaaat cagccgaaaa catacgttca gcacgtaatg    2940
   gaacaagacg gcaagaaatt gattgaactt cttgatcaag gagcgcactt ctatatttgc    3000
   ggagacggaa gccaaatggc acctgccgtt gaagcaacgc ttatgaaaag ctatgctgac    3060
   gttcaccaag tgagtgaagc agacgctcgc ttatggctgc agcagctaga agaaaaaggc    3120
   cgatacgcaa aagacgtgtg ggctgggtaa                                     3150
<210> 48
   <211> 3150
   <212> DNA
   <213> Artificial Sequence
<220>
   <223> P450BM3 N320P
<400> 48
   atgacaatta aagaaatgcc tcagccaaaa acgtttggag agcttaaaaa tttaccgtta      60
   ttaaacacag ataaaccggt tcaagctttg atgaaaattg cggatgaatt aggagaaatc     120
   tttaaattcg aggcgcctgg tcgtgtaacg cgctacttat caagtcagcg tctaattaaa     180
   gaagcatgcg atgaatcacg ctttgataaa aacttaagtc aagcgcttaa atttgtacgt     240
   gattttgcag gagacgggtt atttacaagc tggacgcatg aaaaaaattg gaaaaaagcg     300
   cataatatct tacttccaag cttcagtcag caggcaatga aaggctatca tgcgatgatg 360
   gtcgatatcg ccgtgcagct tgttcaaaag tgggagcgtc taaatgcaga tgagcatatt 420
   gaagtaccgg aagacatgac acgtttaacg cttgatacaa ttggtctttg cggctttaac 480
   tatcgcttta acagctttta ccgagatcag cctcatccat ttattacaag tatggtccgt 540
   gcactggatg aagcaatgaa caagctgcag cgagcaaatc cagacgaccc agcttatgat 600
   gaaaacaagc gccagtttca agaagatatc aaggtgatga acgacctagt agataaaatt 660
   attgcagatc gcaaagcaag cggtgaacaa agcgatgatt tattaacgca tatgctaaac 720
   ggaaaagatc cagaaacggg tgagccgctt gatgacgaga acattcgcta tcaaattatt 780
   acattcttaa ttgcgggaca cgaaacaaca agtggtcttt tatcatttgc gctgtatttc 840
   ttagtgaaaa atccacatgt attacaaaaa gcagcagaag aagcagcacg agttctagta 900
   gatcctgttc caagctacaa acaagtcaaa cagcttaaat atgtcggcat ggtcttaccg 960
   gaagcgctgc gcttatggcc aactgctcct gcgttttccc tatatgcaaa agaagatacg 1020
   gtgcttggag gagaatatcc tttagaaaaa ggcgacgaac taatggttct gattcctcag 1080
   cttcaccgtg ataaaacaat ttggggagac gatgtggaag agttccgtcc agagcgtttt 1140
   gaaaatccaa gtgcgattcc gcagcatgcg tttaaaccgt ttggaaacgg tcagcgtgcg 1200
   tgtatcggtc agcagttcgc tcttcatgaa gcaacgctgg tacttggtat gatgctaaaa 1260
   cactttgact ttgaagatca tacaaactac gagctggata ttaaagaaac tttaacgtta 1320
   aaacctgaag gctttgtggt aaaagcaaaa tcgaaaaaaa ttccgcttgg cggtattcct 1380
   tcacctagca ctgaacagtc tgctaaaaaa gtacgcaaaa aggcagaaaa cgctcataat 1440
   acgccgctgc ttgtgctata cggttcaaat atgggaacag ctgaaggaac ggcgcgtgat 1500
   ttagcagata ttgcaatgag caaaggattt gcaccgcagg tcgcaacgct tgattcacac 1560
   gccggaaatc ttccgcgcga aggagctgta ttaattgtaa cggcgtctta taacggtcat 1620
   ccgcctgata acgcaaagca atttgtcgac tggttagacc aagcgtctgc tgatgaagta 1680
   aaaggcgttc gctactccgt atttggatgc ggcgataaaa actgggctac tacgtatcaa 1740
   aaagtgcctg cttttatcga tgaaacgctt gccgctaaag gggcagaaaa catcgctgac 1800
   cgcggtgaag cagatgcaag cgacgacttt gaaggcacat atgaagaatg gcgtgaacat 1860
   atgtggagtg acgtagcagc ctactttaac ctcgacattg aaaacagtga agataataaa 1920
   tctactcttt cacttcaatt tgtcgacagc gccgcggata tgccgcttgc gaaaatgcac 1980
   ggtgcgtttt caacgaacgt cgtagcaagc aaagaacttc aacagccagg cagtgcacga 2040
   agcacgcgac atcttgaaat tgaacttcca aaagaagctt cttatcaaga aggagatcat 2100
   ttaggtgtta ttcctcgcaa ctatgaagga atagtaaacc gtgtaacagc aaggttcggc 2160
   ctagatgcat cacagcaaat ccgtctggaa gcagaagaag aaaaattagc tcatttgcca 2220
   ctcgctaaaa cagtatccgt agaagagctt ctgcaatacg tggagcttca agatcctgtt    2280
   acgcgcacgc agcttcgcgc aatggctgct aaaacggtct gcccgccgca taaagtagag    2340
   cttgaagcct tgcttgaaaa gcaagcctac aaagaacaag tgctggcaaa acgtttaaca    2400
   atgcttgaac tgcttgaaaa atacccggcg tgtgaaatga aattcagcga atttatcgcc    2460
   cttctgccaa gcatacgccc gcgctattac tcgatttctt catcacctcg tgtcgatgaa    2520
   aaacaagcaa gcatcacggt cagcgttgtc tcaggagaag cgtggagcgg atatggagaa    2580
   tataaaggaa ttgcgtcgaa ctatcttgcc gagctgcaag aaggagatac gattacgtgc    2640
   tttatttcca caccgcagtc agaatttacg ctgccaaaag accctgaaac gccgcttatc    2700
   atggtcggac cgggaacagg cgtcgcgccg tttagaggct ttgtgcaggc gcgcaaacag    2760
   ctaaaagaac aaggacagtc acttggagaa gcacatttat acttcggctg ccgttcacct    2820
   catgaagact atctgtatca agaagagctt gaaaacgccc aaagcgaagg catcattacg    2880
   cttcataccg ctttttctcg catgccaaat cagccgaaaa catacgttca gcacgtaatg    2940
   gaacaagacg gcaagaaatt gattgaactt cttgatcaag gagcgcactt ctatatttgc    3000
   ggagacggaa gccaaatggc acctgccgtt gaagcaacgc ttatgaaaag ctatgctgac    3060
   gttcaccaag tgagtgaagc agacgctcgc ttatggctgc agcagctaga agaaaaaggc    3120
   cgatacgcaa aagacgtgtg ggctgggtaa                                     3150
<210> 49
   <211> 3150
   <212> DNA
   <213> Artificial Sequence
<220>
   <223> P450BM3 N320S
<400> 49
   atgacaatta aagaaatgcc tcagccaaaa acgtttggag agcttaaaaa tttaccgtta        60
   ttaaacacag ataaaccggt tcaagctttg atgaaaattg cggatgaatt aggagaaatc       120
   tttaaattcg aggcgcctgg tcgtgtaacg cgctacttat caagtcagcg tctaattaaa       180
   gaagcatgcg atgaatcacg ctttgataaa aacttaagtc aagcgcttaa atttgtacgt       240
   gattttgcag gagacgggtt atttacaagc tggacgcatg aaaaaaattg gaaaaaagcg       300
   cataatatct tacttccaag cttcagtcag caggcaatga aaggctatca tgcgatgatg       360
   gtcgatatcg ccgtgcagct tgttcaaaag tgggagcgtc taaatgcaga tgagcatatt       420
   gaagtaccgg aagacatgac acgtttaacg cttgatacaa ttggtctttg cggctttaac       480
   tatcgcttta acagctttta ccgagatcag cctcatccat ttattacaag tatggtccgt       540
   gcactggatg aagcaatgaa caagctgcag cgagcaaatc cagacgaccc agcttatgat       600
   gaaaacaagc gccagtttca agaagatatc aaggtgatga acgacctagt agataaaatt 660
   attgcagatc gcaaagcaag cggtgaacaa agcgatgatt tattaacgca tatgctaaac 720
   ggaaaagatc cagaaacggg tgagccgctt gatgacgaga acattcgcta tcaaattatt 780
   acattcttaa ttgcgggaca cgaaacaaca agtggtcttt tatcatttgc gctgtatttc 840
   ttagtgaaaa atccacatgt attacaaaaa gcagcagaag aagcagcacg agttctagta 900
   gatcctgttc caagctacaa acaagtcaaa cagcttaaat atgtcggcat ggtcttaagc 960
   gaagcgctgc gcttatggcc aactgctcct gcgttttccc tatatgcaaa agaagatacg 1020
   gtgcttggag gagaatatcc tttagaaaaa ggcgacgaac taatggttct gattcctcag 1080
   cttcaccgtg ataaaacaat ttggggagac gatgtggaag agttccgtcc agagcgtttt 1140
   gaaaatccaa gtgcgattcc gcagcatgcg tttaaaccgt ttggaaacgg tcagcgtgcg 1200
   tgtatcggtc agcagttcgc tcttcatgaa gcaacgctgg tacttggtat gatgctaaaa 1260
   cactttgact ttgaagatca tacaaactac gagctggata ttaaagaaac tttaacgtta 1320
   aaacctgaag gctttgtggt aaaagcaaaa tcgaaaaaaa ttccgcttgg cggtattcct 1380
   tcacctagca ctgaacagtc tgctaaaaaa gtacgcaaaa aggcagaaaa cgctcataat 1440
   acgccgctgc ttgtgctata cggttcaaat atgggaacag ctgaaggaac ggcgcgtgat 1500
   ttagcagata ttgcaatgag caaaggattt gcaccgcagg tcgcaacgct tgattcacac 1560
   gccggaaatc ttccgcgcga aggagctgta ttaattgtaa cggcgtctta taacggtcat 1620
   ccgcctgata acgcaaagca atttgtcgac tggttagacc aagcgtctgc tgatgaagta 1680
   aaaggcgttc gctactccgt atttggatgc ggcgataaaa actgggctac tacgtatcaa 1740
   aaagtgcctg cttttatcga tgaaacgctt gccgctaaag gggcagaaaa catcgctgac 1800
   cgcggtgaag cagatgcaag cgacgacttt gaaggcacat atgaagaatg gcgtgaacat 1860
   atgtggagtg acgtagcagc ctactttaac ctcgacattg aaaacagtga agataataaa 1920
   tctactcttt cacttcaatt tgtcgacagc gccgcggata tgccgcttgc gaaaatgcac 1980
   ggtgcgtttt caacgaacgt cgtagcaagc aaagaacttc aacagccagg cagtgcacga 2040
   agcacgcgac atcttgaaat tgaacttcca aaagaagctt cttatcaaga aggagatcat 2100
   ttaggtgtta ttcctcgcaa ctatgaagga atagtaaacc gtgtaacagc aaggttcggc 2160
   ctagatgcat cacagcaaat ccgtctggaa gcagaagaag aaaaattagc tcatttgcca 2220
   ctcgctaaaa cagtatccgt agaagagctt ctgcaatacg tggagcttca agatcctgtt 2280
   acgcgcacgc agcttcgcgc aatggctgct aaaacggtct gcccgccgca taaagtagag 2340
   cttgaagcct tgcttgaaaa gcaagcctac aaagaacaag tgctggcaaa acgtttaaca 2400
   atgcttgaac tgcttgaaaa atacccggcg tgtgaaatga aattcagcga atttatcgcc 2460
   cttctgccaa gcatacgccc gcgctattac tcgatttctt catcacctcg tgtcgatgaa 2520
   aaacaagcaa gcatcacggt cagcgttgtc tcaggagaag cgtggagcgg atatggagaa 2580
   tataaaggaa ttgcgtcgaa ctatcttgcc gagctgcaag aaggagatac gattacgtgc 2640
   tttatttcca caccgcagtc agaatttacg ctgccaaaag accctgaaac gccgcttatc 2700
   atggtcggac cgggaacagg cgtcgcgccg tttagaggct ttgtgcaggc gcgcaaacag 2760
   ctaaaagaac aaggacagtc acttggagaa gcacatttat acttcggctg ccgttcacct 2820
   catgaagact atctgtatca agaagagctt gaaaacgccc aaagcgaagg catcattacg 2880
   cttcataccg ctttttctcg catgccaaat cagccgaaaa catacgttca gcacgtaatg 2940
   gaacaagacg gcaagaaatt gattgaactt cttgatcaag gagcgcactt ctatatttgc 3000
   ggagacggaa gccaaatggc acctgccgtt gaagcaacgc ttatgaaaag ctatgctgac 3060
   gttcaccaag tgagtgaagc agacgctcgc ttatggctgc agcagctaga agaaaaaggc 3120
   cgatacgcaa aagacgtgtg ggctgggtaa 3150
<210> 50
   <211> 3150
   <212> DNA
   <213> Artificial Sequence
<220>
   <223> P450BM3 N320E
<400> 50
   atgacaatta aagaaatgcc tcagccaaaa acgtttggag agcttaaaaa tttaccgtta      60
   ttaaacacag ataaaccggt tcaagctttg atgaaaattg cggatgaatt aggagaaatc     120
   tttaaattcg aggcgcctgg tcgtgtaacg cgctacttat caagtcagcg tctaattaaa     180
   gaagcatgcg atgaatcacg ctttgataaa aacttaagtc aagcgcttaa atttgtacgt     240
   gattttgcag gagacgggtt atttacaagc tggacgcatg aaaaaaattg gaaaaaagcg     300
   cataatatct tacttccaag cttcagtcag caggcaatga aaggctatca tgcgatgatg     360
   gtcgatatcg ccgtgcagct tgttcaaaag tgggagcgtc taaatgcaga tgagcatatt     420
   gaagtaccgg aagacatgac acgtttaacg cttgatacaa ttggtctttg cggctttaac     480
   tatcgcttta acagctttta ccgagatcag cctcatccat ttattacaag tatggtccgt     540
   gcactggatg aagcaatgaa caagctgcag cgagcaaatc cagacgaccc agcttatgat     600
   gaaaacaagc gccagtttca agaagatatc aaggtgatga acgacctagt agataaaatt     660
   attgcagatc gcaaagcaag cggtgaacaa agcgatgatt tattaacgca tatgctaaac     720
   ggaaaagatc cagaaacggg tgagccgctt gatgacgaga acattcgcta tcaaattatt     780
   acattcttaa ttgcgggaca cgaaacaaca agtggtcttt tatcatttgc gctgtatttc     840
   ttagtgaaaa atccacatgt attacaaaaa gcagcagaag aagcagcacg agttctagta 900
   gatcctgttc caagctacaa acaagtcaaa cagcttaaat atgtcggcat ggtcttagaa 960
   gaagcgctgc gcttatggcc aactgctcct gcgttttccc tatatgcaaa agaagatacg 1020
   gtgcttggag gagaatatcc tttagaaaaa ggcgacgaac taatggttct gattcctcag 1080
   cttcaccgtg ataaaacaat ttggggagac gatgtggaag agttccgtcc agagcgtttt 1140
   gaaaatccaa gtgcgattcc gcagcatgcg tttaaaccgt ttggaaacgg tcagcgtgcg 1200
   tgtatcggtc agcagttcgc tcttcatgaa gcaacgctgg tacttggtat gatgctaaaa 1260
   cactttgact ttgaagatca tacaaactac gagctggata ttaaagaaac tttaacgtta 1320
   aaacctgaag gctttgtggt aaaagcaaaa tcgaaaaaaa ttccgcttgg cggtattcct 1380
   tcacctagca ctgaacagtc tgctaaaaaa gtacgcaaaa aggcagaaaa cgctcataat 1440
   acgccgctgc ttgtgctata cggttcaaat atgggaacag ctgaaggaac ggcgcgtgat 1500
   ttagcagata ttgcaatgag caaaggattt gcaccgcagg tcgcaacgct tgattcacac 1560
   gccggaaatc ttccgcgcga aggagctgta ttaattgtaa cggcgtctta taacggtcat 1620
   ccgcctgata acgcaaagca atttgtcgac tggttagacc aagcgtctgc tgatgaagta 1680
   aaaggcgttc gctactccgt atttggatgc ggcgataaaa actgggctac tacgtatcaa 1740
   aaagtgcctg cttttatcga tgaaacgctt gccgctaaag gggcagaaaa catcgctgac 1800
   cgcggtgaag cagatgcaag cgacgacttt gaaggcacat atgaagaatg gcgtgaacat 1860
   atgtggagtg acgtagcagc ctactttaac ctcgacattg aaaacagtga agataataaa 1920
   tctactcttt cacttcaatt tgtcgacagc gccgcggata tgccgcttgc gaaaatgcac 1980
   ggtgcgtttt caacgaacgt cgtagcaagc aaagaacttc aacagccagg cagtgcacga 2040
   agcacgcgac atcttgaaat tgaacttcca aaagaagctt cttatcaaga aggagatcat 2100
   ttaggtgtta ttcctcgcaa ctatgaagga atagtaaacc gtgtaacagc aaggttcggc 2160
   ctagatgcat cacagcaaat ccgtctggaa gcagaagaag aaaaattagc tcatttgcca 2220
   ctcgctaaaa cagtatccgt agaagagctt ctgcaatacg tggagcttca agatcctgtt 2280
   acgcgcacgc agcttcgcgc aatggctgct aaaacggtct gcccgccgca taaagtagag 2340
   cttgaagcct tgcttgaaaa gcaagcctac aaagaacaag tgctggcaaa acgtttaaca 2400
   atgcttgaac tgcttgaaaa atacccggcg tgtgaaatga aattcagcga atttatcgcc 2460
   cttctgccaa gcatacgccc gcgctattac tcgatttctt catcacctcg tgtcgatgaa 2520
   aaacaagcaa gcatcacggt cagcgttgtc tcaggagaag cgtggagcgg atatggagaa 2580
   tataaaggaa ttgcgtcgaa ctatcttgcc gagctgcaag aaggagatac gattacgtgc 2640
   tttatttcca caccgcagtc agaatttacg ctgccaaaag accctgaaac gccgcttatc 2700
   atggtcggac cgggaacagg cgtcgcgccg tttagaggct ttgtgcaggc gcgcaaacag 2760
   ctaaaagaac aaggacagtc acttggagaa gcacatttat acttcggctg ccgttcacct        2820
   catgaagact atctgtatca agaagagctt gaaaacgccc aaagcgaagg catcattacg        2880
   cttcataccg ctttttctcg catgccaaat cagccgaaaa catacgttca gcacgtaatg        2940
   gaacaagacg gcaagaaatt gattgaactt cttgatcaag gagcgcactt ctatatttgc        3000
   ggagacggaa gccaaatggc acctgccgtt gaagcaacgc ttatgaaaag ctatgctgac        3060
   gttcaccaag tgagtgaagc agacgctcgc ttatggctgc agcagctaga agaaaaaggc        3120
   cgatacgcaa aagacgtgtg ggctgggtaa                                          3150
<210> 51
   <211> 1248
   <212> DNA
   <213> Artificial Sequence
<220>
   <223> CamC F351Y
<400> 51
   atgacgactg aaaccataca aagcaacgcc aatcttgccc ctctgccacc ccatgtgcca     60
   gagcacctgg tattcgactt cgacatgtac aatccgtcga atctgtctgc cggcgtgcag    120
   gaggcctggg cagttctgca agaatcaaac gtaccggatc tggtgtggac tcgctgcaac    180
   ggcggacact ggatcgccac tcgcggccaa ctgatccgtg aggcctatga agattaccgc    240
   cacttttcca gcgagtgccc gttcatccct cgtgaagccg gcgaagccta cgacttcatt    300
   cccacctcga tggatccgcc cgagcagcgc cagtttcgtg cgctggccaa ccaagtggtt    360
   ggcatgccgg tggtggataa gctggagaac cggatccagg agctggcctg ctcgctgatc    420
   gagagcctgc gcccgcaagg acagtgcaac ttcaccgagg actacgccga acccttcccg    480
   atacgcatct tcatgctgct cgcaggtcta ccggaagaag atatcccgca cttgaaatac    540
   ctaacggatc agatgacccg tccggatggc agcatgacct tcgcagaggc caaggaggcg    600
   ctctacgact atctgatacc gatcatcgag caacgcaggc agaagccggg aaccgacgct    660
   atcagcatcg ttgccaacgg ccaggtcaat gggcgaccga tcaccagtga cgaagccaag    720
   aggatgtgtg gcctgttact ggtcggcggc ctggatacgg tggtcaattt cctcagcttc    780
   agcatggagt tcctggccaa aagcccggag catcgccagg agctgatcga gcgtcccgag    840
   cgtattccag ccgcttgcga ggaactactc cggcgcttct cgctggttgc cgatggccgc    900
   atcctcacct ccgattacga gtttcatggc gtgcaactga agaaaggtga ccagatcctg    960
   ctaccgcaga tgctgtctgg cctggatgag cgcgaaaacg cctgcccgat gcacgtcgac   1020
   ttcagtcgcc aaaaggtttc acacaccacc tatggccacg gcagccatct gtgccttggc   1080
   cagcacctgg cccgccggga aatcatcgtc accctcaagg aatggctgac caggattcct   1140
   gacttctcca ttgccccggg tgcccagatt cagcacaaga gcggcatcgt cagcggcgtg   1200
   caggcactcc ctctggtctg ggatccggcg actaccaaag cggtataa                 1248
<210> 52
   <211> 1248
   <212> DNA
   <213> Artificial Sequence
<220>
   <223> CamC F351T
<400> 52
   atgacgactg aaaccataca aagcaacgcc aatcttgccc ctctgccacc ccatgtgcca        60
   gagcacctgg tattcgactt cgacatgtac aatccgtcga atctgtctgc cggcgtgcag       120
   gaggcctggg cagttctgca agaatcaaac gtaccggatc tggtgtggac tcgctgcaac       180
   ggcggacact ggatcgccac tcgcggccaa ctgatccgtg aggcctatga agattaccgc       240
   cacttttcca gcgagtgccc gttcatccct cgtgaagccg gcgaagccta cgacttcatt       300
   cccacctcga tggatccgcc cgagcagcgc cagtttcgtg cgctggccaa ccaagtggtt       360
   ggcatgccgg tggtggataa gctggagaac cggatccagg agctggcctg ctcgctgatc       420
   gagagcctgc gcccgcaagg acagtgcaac ttcaccgagg actacgccga acccttcccg       480
   atacgcatct tcatgctgct cgcaggtcta ccggaagaag atatcccgca cttgaaatac       540
   ctaacggatc agatgacccg tccggatggc agcatgacct tcgcagaggc caaggaggcg       600
   ctctacgact atctgatacc gatcatcgag caacgcaggc agaagccggg aaccgacgct       660
   atcagcatcg ttgccaacgg ccaggtcaat gggcgaccga tcaccagtga cgaagccaag       720
   aggatgtgtg gcctgttact ggtcggcggc ctggatacgg tggtcaattt cctcagcttc       780
   agcatggagt tcctggccaa aagcccggag catcgccagg agctgatcga gcgtcccgag       840
   cgtattccag ccgcttgcga ggaactactc cggcgcttct cgctggttgc cgatggccgc       900
   atcctcacct ccgattacga gtttcatggc gtgcaactga agaaaggtga ccagatcctg       960
   ctaccgcaga tgctgtctgg cctggatgag cgcgaaaacg cctgcccgat gcacgtcgac      1020
   ttcagtcgcc aaaaggtttc acacaccacc accggccacg gcagccatct gtgccttggc      1080
   cagcacctgg cccgccggga aatcatcgtc accctcaagg aatggctgac caggattcct      1140
   gacttctcca ttgccccggg tgcccagatt cagcacaaga gcggcatcgt cagcggcgtg      1200
   caggcactcc ctctggtctg ggatccggcg actaccaaag cggtataa                   1248
<210> 53
   <211> 1248
   <212> DNA
   <213> Artificial Sequence
<220>
   <223> CamC F351N
<400> 53
   atgacgactg aaaccataca aagcaacgcc aatcttgccc ctctgccacc ccatgtgcca      60
   gagcacctgg tattcgactt cgacatgtac aatccgtcga atctgtctgc cggcgtgcag     120
   gaggcctggg cagttctgca agaatcaaac gtaccggatc tggtgtggac tcgctgcaac     180
   ggcggacact ggatcgccac tcgcggccaa ctgatccgtg aggcctatga agattaccgc     240
   cacttttcca gcgagtgccc gttcatccct cgtgaagccg gcgaagccta cgacttcatt     300
   cccacctcga tggatccgcc cgagcagcgc cagtttcgtg cgctggccaa ccaagtggtt     360
   ggcatgccgg tggtggataa gctggagaac cggatccagg agctggcctg ctcgctgatc     420
   gagagcctgc gcccgcaagg acagtgcaac ttcaccgagg actacgccga acccttcccg     480
   atacgcatct tcatgctgct cgcaggtcta ccggaagaag atatcccgca cttgaaatac     540
   ctaacggatc agatgacccg tccggatggc agcatgacct tcgcagaggc caaggaggcg     600
   ctctacgact atctgatacc gatcatcgag caacgcaggc agaagccggg aaccgacgct     660
   atcagcatcg ttgccaacgg ccaggtcaat gggcgaccga tcaccagtga cgaagccaag     720
   aggatgtgtg gcctgttact ggtcggcggc ctggatacgg tggtcaattt cctcagcttc     780
   agcatggagt tcctggccaa aagcccggag catcgccagg agctgatcga gcgtcccgag     840
   cgtattccag ccgcttgcga ggaactactc cggcgcttct cgctggttgc cgatggccgc     900
   atcctcacct ccgattacga gtttcatggc gtgcaactga agaaaggtga ccagatcctg     960
   ctaccgcaga tgctgtctgg cctggatgag cgcgaaaacg cctgcccgat gcacgtcgac    1020
   ttcagtcgcc aaaaggtttc acacaccacc aacggccacg gcagccatct gtgccttggc    1080
   cagcacctgg cccgccggga aatcatcgtc accctcaagg aatggctgac caggattcct    1140
   gacttctcca ttgccccggg tgcccagatt cagcacaaga gcggcatcgt cagcggcgtg    1200
   caggcactcc ctctggtctg ggatccggcg actaccaaag cggtataa                 1248
<210> 54
   <211> 1248
   <212> DNA
   <213> Artificial Sequence
<220>
   <223> CamC F351Q
<400> 54
   atgacgactg aaaccataca aagcaacgcc aatcttgccc ctctgccacc ccatgtgcca      60
   gagcacctgg tattcgactt cgacatgtac aatccgtcga atctgtctgc cggcgtgcag     120
   gaggcctggg cagttctgca agaatcaaac gtaccggatc tggtgtggac tcgctgcaac 180
   ggcggacact ggatcgccac tcgcggccaa ctgatccgtg aggcctatga agattaccgc 240
   cacttttcca gcgagtgccc gttcatccct cgtgaagccg gcgaagccta cgacttcatt 300
   cccacctcga tggatccgcc cgagcagcgc cagtttcgtg cgctggccaa ccaagtggtt 360
   ggcatgccgg tggtggataa gctggagaac cggatccagg agctggcctg ctcgctgatc 420
   gagagcctgc gcccgcaagg acagtgcaac ttcaccgagg actacgccga acccttcccg 480
   atacgcatct tcatgctgct cgcaggtcta ccggaagaag atatcccgca cttgaaatac 540
   ctaacggatc agatgacccg tccggatggc agcatgacct tcgcagaggc caaggaggcg 600
   ctctacgact atctgatacc gatcatcgag caacgcaggc agaagccggg aaccgacgct 660
   atcagcatcg ttgccaacgg ccaggtcaat gggcgaccga tcaccagtga cgaagccaag 720
   aggatgtgtg gcctgttact ggtcggcggc ctggatacgg tggtcaattt cctcagcttc 780
   agcatggagt tcctggccaa aagcccggag catcgccagg agctgatcga gcgtcccgag 840
   cgtattccag ccgcttgcga ggaactactc cggcgcttct cgctggttgc cgatggccgc 900
   atcctcacct ccgattacga gtttcatggc gtgcaactga agaaaggtga ccagatcctg 960
   ctaccgcaga tgctgtctgg cctggatgag cgcgaaaacg cctgcccgat gcacgtcgac 1020
   ttcagtcgcc aaaaggtttc acacaccacc cagggccacg gcagccatct gtgccttggc 1080
   cagcacctgg cccgccggga aatcatcgtc accctcaagg aatggctgac caggattcct 1140
   gacttctcca ttgccccggg tgcccagatt cagcacaaga gcggcatcgt cagcggcgtg 1200
   caggcactcc ctctggtctg ggatccggcg actaccaaag cggtataa 1248
<210> 55
   <211> 1248
   <212> DNA
   <213> Artificial Sequence
<220>
   <223> CamC F351H
<400> 55
   atgacgactg aaaccataca aagcaacgcc aatcttgccc ctctgccacc ccatgtgcca 60
   gagcacctgg tattcgactt cgacatgtac aatccgtcga atctgtctgc cggcgtgcag 120
   gaggcctggg cagttctgca agaatcaaac gtaccggatc tggtgtggac tcgctgcaac 180
   ggcggacact ggatcgccac tcgcggccaa ctgatccgtg aggcctatga agattaccgc 240
   cacttttcca gcgagtgccc gttcatccct cgtgaagccg gcgaagccta cgacttcatt 300
   cccacctcga tggatccgcc cgagcagcgc cagtttcgtg cgctggccaa ccaagtggtt 360
   ggcatgccgg tggtggataa gctggagaac cggatccagg agctggcctg ctcgctgatc 420
   gagagcctgc gcccgcaagg acagtgcaac ttcaccgagg actacgccga acccttcccg 480
   atacgcatct tcatgctgct cgcaggtcta ccggaagaag atatcccgca cttgaaatac 540
   ctaacggatc agatgacccg tccggatggc agcatgacct tcgcagaggc caaggaggcg 600
   ctctacgact atctgatacc gatcatcgag caacgcaggc agaagccggg aaccgacgct 660
   atcagcatcg ttgccaacgg ccaggtcaat gggcgaccga tcaccagtga cgaagccaag 720
   aggatgtgtg gcctgttact ggtcggcggc ctggatacgg tggtcaattt cctcagcttc 780
   agcatggagt tcctggccaa aagcccggag catcgccagg agctgatcga gcgtcccgag 840
   cgtattccag ccgcttgcga ggaactactc cggcgcttct cgctggttgc cgatggccgc 900
   atcctcacct ccgattacga gtttcatggc gtgcaactga agaaaggtga ccagatcctg 960
   ctaccgcaga tgctgtctgg cctggatgag cgcgaaaacg cctgcccgat gcacgtcgac 1020
   ttcagtcgcc aaaaggtttc acacaccacc catggccacg gcagccatct gtgccttggc 1080
   cagcacctgg cccgccggga aatcatcgtc accctcaagg aatggctgac caggattcct 1140
   gacttctcca ttgccccggg tgcccagatt cagcacaaga gcggcatcgt cagcggcgtg 1200
   caggcactcc ctctggtctg ggatccggcg actaccaaag cggtataa 1248
<210> 56
   <211> 1248
   <212> DNA
   <213> Artificial Sequence
<220>
   <223> CamC F351D
<400> 56
   atgacgactg aaaccataca aagcaacgcc aatcttgccc ctctgccacc ccatgtgcca         60
   gagcacctgg tattcgactt cgacatgtac aatccgtcga atctgtctgc cggcgtgcag        120
   gaggcctggg cagttctgca agaatcaaac gtaccggatc tggtgtggac tcgctgcaac        180
   ggcggacact ggatcgccac tcgcggccaa ctgatccgtg aggcctatga agattaccgc        240
   cacttttcca gcgagtgccc gttcatccct cgtgaagccg gcgaagccta cgacttcatt        300
   cccacctcga tggatccgcc cgagcagcgc cagtttcgtg cgctggccaa ccaagtggtt        360
   ggcatgccgg tggtggataa gctggagaac cggatccagg agctggcctg ctcgctgatc        420
   gagagcctgc gcccgcaagg acagtgcaac ttcaccgagg actacgccga acccttcccg        480
   atacgcatct tcatgctgct cgcaggtcta ccggaagaag atatcccgca cttgaaatac        540
   ctaacggatc agatgacccg tccggatggc agcatgacct tcgcagaggc caaggaggcg        600
   ctctacgact atctgatacc gatcatcgag caacgcaggc agaagccggg aaccgacgct        660
   atcagcatcg ttgccaacgg ccaggtcaat gggcgaccga tcaccagtga cgaagccaag        720
   aggatgtgtg gcctgttact ggtcggcggc ctggatacgg tggtcaattt cctcagcttc        780
   agcatggagt tcctggccaa aagcccggag catcgccagg agctgatcga gcgtcccgag        840
   cgtattccag ccgcttgcga ggaactactc cggcgcttct cgctggttgc cgatggccgc        900
   atcctcacct ccgattacga gtttcatggc gtgcaactga agaaaggtga ccagatcctg        960
   ctaccgcaga tgctgtctgg cctggatgag cgcgaaaacg cctgcccgat gcacgtcgac       1020
   ttcagtcgcc aaaaggtttc acacaccacc gatggccacg gcagccatct gtgccttggc       1080
   cagcacctgg cccgccggga aatcatcgtc accctcaagg aatggctgac caggattcct       1140
   gacttctcca ttgccccggg tgcccagatt cagcacaaga gcggcatcgt cagcggcgtg       1200
   caggcactcc ctctggtctg ggatccggcg actaccaaag cggtataa                    1248